# EUROPEAN PATENT APPLICATION

(11) **EP 4 541 897 A2**
(43) Date of publication of application: **23.04.2025**
(21) Application number: 25152717.2
(22) Date of filing: 24.06.2020
(51) Int. Cl.: C12N 15/63

(54) **MAMMALIAN CELL LINES WITH SIRT-1 GENE KNOCKOUT**

(30) Priority: 26.06.2019 EP 19182558
(62) Divisional of application: 20733822.9
(71) Applicant: F. Hoffmann-La Roche AG, 4070 Basel (CH)
(72) Inventor: AUSLAENDER, Simon, 82377 Penzberg (DE); OSWALD, Benedikt, 82377 Penzberg (DE)
(74) Representative: Meier, Kerstin

(57) **Abstract**

Herein is reported a method for generating a recombinant mammalian cell expressing a heterologous polypeptide and a method for producing a heterologous polypeptide using said recombinant mammalian cell, wherein in the recombinant cell the expression of the endogenous SIRT-1 gene has been reduced. It has been found that the knockout of the sirtuin-1 gene (SIRT-1) in mammalian cells, e.g. such as CHO cells, improves recombinant productivity and reduces lactate production by the cells. Additionally, it has been found that the viability decline at the end of a fed-batch fermentation is reduced.

## Description

The current invention is in the field of cell line development for the recombinant production of therapeutic polypeptides, such as therapeutic antibodies. In more detail, herein is reported a mammalian cell with a functional knock-out of the SIRT-1 gene, which results in improved expression characteristics.

### Background

Mammalian host cell lines, especially CHO and HEK cell lines, are used for the recombinant production of secreted proteins, such as supply proteins (e.g. antigens, receptors and others) and therapeutic molecules (e.g. antibodies, cytokines and others). These host cell lines are transfected with vectors comprising the expression cassettes encoding the corresponding therapeutic molecule. Subsequently stable transfectants are selected by applying selective pressure. This results in a cell pool consisting of individual clones. In a single cell cloning step, these clones are isolated and subsequently screened with different assays to identify top producer cells.

Genetic engineering approaches have been applied to host cell lines in order to improve their characteristics, such as (i) overexpression of endogenous proteins involved in the unfolded protein response pathway to improve protein folding and secretion (Gulis, G., et al., BMC biotechnology, 14 (2014) 26), (ii) overexpression of anti-apoptotic proteins to improve cell viability and prolong the fermentation process (Lee, J. S., et al., Biotechnol. Bioeng. 110 (2013) 2195-2207), (iii) overexpression of miRNA and/or shRNA molecules to improve cell growth and productivity (Fischer, S., et al., J. Biotechnol. 212 (2015) 32-43), (iv) overexpression of glycoenzymes to modulate glycosylation pattern of therapeutic molecules (Ferrara, C., et al., Biotechnol. Bioeng. 93 (2006) 851-861) and many others (Fischer, S., et al., Biotechnol. Adv. 33 (2015) 1878-1896).

In addition, knockout of endogenous proteins has been shown to improve cell characteristics. Examples are (i) knockout of BAX/BAK proteins leading to increased apoptosis resistance (Cost, G. J., et al., Biotechnol. Bioeng. 105 (2010) 330-340), (ii) knockout of PUTS to produce non-fucosylated proteins (Yamane-Ohnuki, N., et al., Biotechnol. Bioeng. 87 (2004) 614-622), (iii) knockout of GS to increase selection efficiency using GS selection system (Fan, L., et al., Biotechnol. Bioeng. 109 (2012) 1007-1015) and many others (Fischer, S., et al., Biotechnol. Adv. 33 (2015) 1878-1896). While zinc finger or TALEN proteins are mainly used in the past, CRISPR/Cas9 recently has been established for versatile and simple targeting of genomic sequences for knockout purposes. For example, miRNA-744 was targeted in CHO cells using CRISPR/Cas9 by using multiple gRNA enabling sequence excision (Raab, N., et al., Biotechnol. J. (2019) 1800477).

CN 109 161 545 discloses a microRNA for inhibiting expression of SIRT-1 of chicken, and also discloses a recombinant over-expression plasmid and specific application of the microRNA, and an LMH cell line for constructing stable low-expression SIRT-1 by utilizing over-expression miRNAs.

US 2007/160586 discloses methods for extending the replicative lifespan of cells.

US 2011/015272 discloses Sirtuin 1 and the treatment of neurodegenerative diseases.

Younghwan, H., et al. disclose the increase of Hspa1a and Hspa1b genes in the resting B cells of SIRT-1 knockout mice (Mol. Biol. Rep. 46 (2019) 4225-4234).

EP 3 308 778 discloses arginine and its use as a t cell modulator.

Fischer, S., et al. disclose enhanced protein production by microRNA-30 family in CHO cells is mediated by the modulation of the ubiquitin pathway (J. Biotechnol. 212 (2015) 32-43).

Currently, there is no knockout of a single endogenous gene known that increases productivity. Thus, a single knockout of an endogenous gene is highly desired because of its simplicity to be introduced in host cell lines.

### Summarv of the Invention

Herein is reported a method for generating a recombinant mammalian cell expressing a heterologous polypeptide and a method for producing a heterologous polypeptide using said recombinant mammalian cell, wherein in the recombinant mammalian cell the activity or function or expression of the endogenous SIRT-1 gene has been reduced or eliminated or diminished or (completely) knocked-out.

The invention is based, at least in part, on the finding that the knockout of the sirtuin-1 (SIRT-1) gene in mammalian cells, e.g. such as CHO cells, improves on the one hand recombinant productivity, e.g. of standard IgG-type antibodies and especially of complex antibody formats, and reduces on the other hand lactate production by the cells during cultivation. Additionally, it has been found that the viability decline at the end of a fed-batch cultivation is reduced for recombinant cells according to the current invention, i.e. the timespan with viability above a certain threshold value is increased, compared to cells with fully functional SIRT-1 gene.

One independent aspect of the current invention is a mammalian cell wherein the activity or/and function or/and expression of the endogenous SIRT-1 gene has been reduced or eliminated or diminished or (completely) knocked-out.

One independent aspect of the current invention is a mammalian cell wherein the expression of the endogenous SIRT-1 gene has been reduced and wherein said mammalian cell has at least one of increased productivity for heterologous polypeptides and/or reduced lactate production during cultivation and/or extended high viability levels during cultivation and/or extended cultivation time compared to a cell cultivated under the same conditions that has the identical genotype but endogenous SIRT-1 gene expression.

One independent aspect of the current invention is a method for at least one of increasing heterologous polypeptide titer and/or reducing lactate production and/or extended high viability levels during cultivation and/or extension of cultivation time of a recombinant mammalian cell having reduced (endogenous) SIRT-1 expression comprising an exogenous nucleic acid encoding said heterologous polypeptide compared to a cell cultivated under the same conditions that has the identical genotype but endogenous SIRT-1 gene expression.

One independent aspect of the current invention is a method for producing a recombinant mammalian cell with improved recombinant productivity and/or reduced lactate production, wherein the method comprises the following steps:
a) applying a nuclease-assisted and/or nucleic acid targeting the endogenous SIRT-1 genes in a mammalian cell to reduce the activity of the endogenous SIRT-1 gene, and
b) selecting a mammalian cell wherein the activity of the endogenous SIRT-1 gene has been reduced,
thereby producing a recombinant mammalian cell with increased recombinant productivity and/or reduced lactate production compared to a compared to a cell cultivated under the same conditions that has the identical genotype but endogenous SIRT-1 gene expression.

One independent aspect of to the current invention is a method for producing a heterologous polypeptide comprising the steps of
a) cultivating a mammalian cell comprising an exogenous deoxyribonucleic acid encoding the heterologous polypeptide optionally under conditions suitable for the expression of the heterologous polypeptide, and
b) recovering the heterologous polypeptide from the cell or the cultivation medium,
wherein the activity or/and function or/and expression of the endogenous SIRT-1 gene has been reduced or eliminated or diminished or (completely) knocked-out.

Another independent aspect of the current invention is a method for producing a recombinant mammalian cell having/with improved and/or increased recombinant productivity and/or reduced lactate production, wherein the method comprises the following steps:
a) applying a nucleic acid or an enzyme or a nuclease-assisted gene targeting system targeting the endogenous SIRT-1 genes to a mammalian cell to reduce or eliminate or diminish or (completely) knock-out the activity or/and function or/and expression of the endogenous SIRT-1 gene, and
b) selecting a mammalian cell wherein the activity or/and function or/and expression of the endogenous SIRT-1 gene has been reduced or eliminated or diminished or (completely) knocked-out,
thereby producing a recombinant mammalian cell having/with improved and/or increased recombinant productivity and/or reduced lactate production

In one embodiment of all aspects and embodiments of the current invention the SIRT-1 gene knockout is a heterozygous knockout or a homozygous knockout.

In one embodiment of all aspects and embodiments of the current invention the productivity of the SIRT-1 knockout cell line is at least 10 %, preferably 15 % or more, most preferred 20 % or more increased compared to a SIRT-1 expressing (parent) mammalian cell.

In one embodiment of all aspects and embodiments of the current invention the reduction or elimination or diminishment or knock-out is mediated by a nuclease-assisted gene targeting system. In one embodiment the nuclease-assisted gene targeting system is selected from the group consisting of CRISPR/Cas9, CRISPR/Cpf1, zinc-finger nuclease and TALEN.

In one embodiment of all aspects and embodiments of the current invention the reduction of SIRT-1 gene expression is mediated by RNA silencing. In one embodiment the RNA silencing is selected from the group consisting of siRNA gene targeting and knock-down, shRNA gene targeting and knock-down, and miRNA gene targeting and knock-down.

In one embodiment of all aspects and embodiments of the current invention the SIRT-1 knockout is performed before the introduction of the exogenous nucleic acid encoding the heterologous polypeptide or after the introduction of the exogenous nucleic acid encoding the heterologous polypeptide.

In one embodiment of all aspects and embodiments of the current invention the polypeptide is an antibody. In one embodiment the antibody is an antibody comprising two or more different binding sites and optionally a domain exchange. In one embodiment the antibody comprises three or more binding sites or VH/VL-pairs or Fab fragments and optionally a domain exchange. In one embodiment the antibody is a multispecific antibody. In one embodiment the multispecific antibody is selected from the group consisting of
i) a full-length antibody with domain exchange comprising a first Fab fragment and a second Fab fragment,
   wherein in the first Fab fragment
   a) the light chain of the first Fab fragment comprises a VL and a CH1 domain and the heavy chain of the first Fab fragment comprises a VH and a CL domain;
   b) the light chain of the first Fab fragment comprises a VH and a CL domain and the heavy chain of the first Fab fragment comprises a VL and a CH1 domain; or
   c) the light chain of the first Fab fragment comprises a VH and a CH1 domain and the heavy chain of the first Fab fragment comprises a VL and a CL domain;
      and
      wherein the second Fab fragment comprises a light chain comprising a VL and a CL domain, and a heavy chain comprising a VH and a CH1 domain;
ii) a full-length antibody with domain exchange and additional heavy chain C-terminal binding site comprising
   - one full length antibody comprising two pairs each of a full length antibody light chain and a full length antibody heavy chain, wherein the binding sites formed by each of the pairs of the full length heavy chain and the full length light chain specifically bind to a first antigen;
      and
   - one additional Fab fragment, wherein the additional Fab fragment is fused to the C-terminus of one heavy chain of the full length antibody, wherein the binding site of the additional Fab fragment specifically binds to a second antigen;
      wherein the additional Fab fragment specifically binding to the second antigen i) comprises a domain crossover such that a) the light chain variable domain (VL) and the heavy chain variable domain (VH) are replaced by each other, or b) the light chain constant domain (CL) and the heavy chain constant domain (CH1) are replaced by each other, or ii) is a single chain Fab fragment;
iii)a one-armed single chain antibody comprising a first binding site that specifically binds to a first epitope or antigen and a second binding site that specifically binds to a second epitope or antigen, comprising
   - a light chain comprising a variable light chain domain and a light chain kappa or lambda constant domain;
   - a combined light/heavy chain comprising a variable light chain domain, a light chain constant domain, a peptidic linker, a variable heavy chain domain, a CH1 domain, a Hinge region, a CH2 domain, and a CH3 with knob mutation;
   - a heavy chain comprising a variable heavy chain domain, a CH1 domain, a hinge region, a CH2 domain, and a CH3 domain with hole mutation;
iv)a two-armed single chain antibody comprising a first binding site that specifically binds to a first epitope or antigen and a second binding site that specifically binds to a second epitope or antigen, comprising
   - a first combined light/heavy chain comprising a variable light chain domain, a light chain constant domain, a peptidic linker, a variable heavy chain domain, a CH1 domain, a Hinge region, a CH2 domain, and a CH3 with hole mutation;
   - a second combined light/heavy chain comprising a variable light chain domain, a light chain constant domain, a peptidic linker, a variable heavy chain domain, a CH1 domain, a Hinge region, a CH2 domain, and a CH3 domain with knob mutation;
v) a common light chain bispecific antibody comprising a first binding site that specifically binds to a first epitope or antigen and a second binding site that specifically binds to a second epitope or antigen, comprising
   - a light chain comprising a variable light chain domain and a light chain constant domain;
   - a first heavy chain comprising a variable heavy chain domain, a CH1 domain, a Hinge region, a CH2 domain, and a CH3 domain with hole mutation;
   - a second heavy chain comprising a variable heavy chain domain, a CH1 domain, a Hinge region, a CH2 domain, and a CH3 domain with knob mutation;
vi) a full-length antibody with additional heavy chain N-terminal binding site with domain exchange comprising
   - a first and a second Fab fragment, wherein each binding site of the first and the second Fab fragment specifically bind to a first antigen;
   - a third Fab fragment, wherein the binding site of the third Fab fragment specifically binds to a second antigen, and wherein the third Fab fragment comprises a domain crossover such that the variable light chain domain (VL) and the variable heavy chain domain (VH) are replaced by each other; and
   - an Fc-region comprising a first Fc-region polypeptide and a second Fc-region polypeptide;
      wherein the first and the second Fab fragment each comprise a heavy chain fragment and a full length light chain,
      wherein the C-terminus of the heavy chain fragment of the first Fab fragment is fused to the N-terminus of the first Fc-region polypeptide,
      wherein the C-terminus of the heavy chain fragment of the second Fab fragment is fused to the N-terminus of the variable light chain domain of the third Fab fragment and the C-terminus of the CH1 domain of the third Fab fragment is fused to the N-terminus of the second Fc-region polypeptide;
         and
vii) an immunoconjugate comprising a full-length antibody and a non-immunoglobulin moiety conjugated to each other optionally via a peptidic linker.

In addition to the various embodiments depicted and claimed, the disclosed subject matter is also directed to other embodiments having other combinations of the features disclosed and claimed herein. As such, the particular features presented herein can be combined with each other in other manners within the scope of the disclosed subject matter such that the disclosed subject matter includes any suitable combination of the features disclosed herein. The description of specific embodiments of the disclosed subject matter has been presented for purposes of illustration and description. It is not intended to be exhaustive or to limit the disclosed subject matter to those embodiments disclosed.

### Detailed Description of Embodiments of the Invention

Herein is reported a method for generating a recombinant mammalian cell expressing a heterologous polypeptide and a method for producing a heterologous polypeptide using said recombinant mammalian cell, wherein in the recombinant cell the activity/function/expression of the endogenous SIRT-1 gene has been reduced/eliminated/diminished/(completely) knocked-out.

The invention is based, at least in part, on the finding that the knockout of the sirtuin-1 (SIRT-1) gene in mammalian cells, e.g. such as CHO cells, improves recombinant productivity, e.g. of standard IgG-type antibodies and especially of complex antibody formats, and reduces lactate production by the cells. Additionally, it has been found that the viability decline at the end of a fed-batch cultivation is reduced.

### I. GENERAL DEFINITIONS

Useful methods and techniques for carrying out the current invention are described in e.g. Ausubel, F.M. (ed.), Current Protocols in Molecular Biology, Volumes I to III (1997); Glover, N.D., and Hames, B.D., ed., DNA Cloning: A Practical Approach, Volumes I and II (1985), Oxford University Press; Freshney, R.I. (ed.), Animal Cell Culture - a practical approach, IRL Press Limited (1986); Watson, J.D., et al., Recombinant DNA, Second Edition, CHSL Press (1992); Winnacker, E.L., From Genes to Clones; N.Y., VCH Publishers (1987); Celis, J., ed., Cell Biology, Second Edition, Academic Press (1998); Freshney, R.I., Culture of Animal Cells: A Manual of Basic Technique, second edition, Alan R. Liss, Inc., N.Y. (1987).

The use of recombinant DNA technology enables the generation of derivatives of a nucleic acid. Such derivatives can, for example, be modified in individual or several nucleotide positions by substitution, alteration, exchange, deletion or insertion. The modification or derivatization can, for example, be carried out by means of site directed mutagenesis. Such modifications can easily be carried out by a person skilled in the art (see e.g. Sambrook, J., et al., Molecular Cloning: A laboratory manual (1999) Cold Spring Harbor Laboratory Press, New York, USA; Hames, B.D., and Higgins, S.G., Nucleic acid hybridization - a practical approach (1985) IRL Press, Oxford, England).

It must be noted that as used herein and in the appended claims, the singular forms "a", "an", and "the" include plural reference unless the context clearly dictates otherwise. Thus, for example, reference to "a cell" includes a plurality of such cells and equivalents thereof known to those skilled in the art, and so forth. As well, the terms "a" (or "an"), "one or more" and "at least one" can be used interchangeably herein. It is also to be noted that the terms "comprising", "including", and "having" can be used interchangeably.

The term "about" denotes a range of +/- 20 % of the thereafter following numerical value. **In** one embodiment the term about denotes a range of +/- 10 % of the thereafter following numerical value. **In** one embodiment the term about denotes a range of +/-5 % of the thereafter following numerical value.

The term "comprising" also encompasses the term "consisting of".

The term "recombinant mammalian cell" as used herein denotes a mammalian cell comprising an exogenous nucleotide sequence capable of expressing a polypeptide. Such recombinant mammalian cells are cells into which one or more exogenous nucleic acid(s) have been introduced, including the progeny of such cells. Thus, the term "a mammalian cell comprising a nucleic acid encoding a heterologous polypeptide" denotes cells comprising an exogenous nucleotide sequence integrated in the genome of the mammalian cell and capable of expressing the heterologous polypeptide. In one embodiment the mammalian cell comprising an exogenous nucleotide sequence is a cell comprising an exogenous nucleotide sequence integrated at a single site within a locus of the genome of the host cell, wherein the exogenous nucleotide sequence comprises a first and a second recombination recognition sequence flanking at least one first selection marker, and a third recombination recognition sequence located between the first and the second recombination recognition sequence, and all the recombination recognition sequences are different

The term "recombinant cell" as used herein denotes a cell after genetic modification, such as, e.g., a cell expressing a heterologous polypeptide of interest and that can be used for the production of said heterologous polypeptide of interest at any scale. For example, "a recombinant mammalian cell comprising an exogenous nucleotide sequence" denotes a cell wherein the coding sequences for a heterologous polypeptide of interest have been introduced into the genome of the host cell. For example, "a recombinant mammalian cell comprising an exogenous nucleotide sequence" that has been subjected to recombinase mediated cassette exchange (RMCE) whereby the coding sequences for a polypeptide of interest have been introduced into the genome of the host cell is a "recombinant cell".

A "mammalian cell comprising an exogenous nucleotide sequence" and a "recombinant cell" are both "transformed cells". This term includes the primary transformed cell as well as progeny derived therefrom without regard to the number of passages. Progeny may, e.g., not be completely identical in nucleic acid content to a parent cell, but may contain mutations. Mutant progeny that has the same function or biological activity as screened or selected for in the originally transformed cell are encompassed.

An "isolated" composition is one which has been separated from a component of its natural environment. In some embodiments, a composition is purified to greater than 95 % or 99 % purity as determined by, for example, electrophoretic (e.g., SDS-PAGE, isoelectric focusing (IEF), capillary electrophoresis, CE-SDS) or chromatographic (e.g., size exclusion chromatography or ion exchange or reverse phase HPLC). For review of methods for assessment of e.g. antibody purity, see, e.g., Flatman, S. et al., J. Chrom. B 848 (2007) 79-87.

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

An "isolated" polypeptide or antibody refers to a polypeptide molecule or antibody molecule that has been separated from a component of its natural environment.

The term "integration site" denotes a nucleic acid sequence within a cell's genome into which an exogenous nucleotide sequence is inserted. In certain embodiments, an integration site is between two adjacent nucleotides in the cell's genome. In certain embodiments, an integration site includes a stretch of nucleotide sequences. In certain embodiments, the integration site is located within a specific locus of the genome of a mammalian cell. In certain embodiments, the integration site is within an endogenous gene of a mammalian cell.

The terms "vector" or "plasmid", which can be used interchangeably, as used herein, refer to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors".

The term "binding to" denotes the binding of a binding site to its target, such as e.g. of an antibody binding site comprising an antibody heavy chain variable domain and an antibody light chain variable domain to the respective antigen. This binding can be determined using, for example, a BIAcore^{®} assay (GE Healthcare, Uppsala, Sweden). That is, the term "binding (to an antigen)" denotes the binding of an antibody in an in vitro assay to its antigen(s). In one embodiment binding is determined in a binding assay in which the antibody is bound to a surface and binding of the antigen to the antibody is measured by Surface Plasmon Resonance (SPR). Binding means e.g. a binding affinity (K_{D}) of 10⁻⁸ M or less, in some embodiments of 10⁻¹³ to 10⁻⁸ M, in some embodiments of 10⁻¹³ to 10⁻⁹ M. The term "binding" also includes the term "specifically binding".

For example, in one possible embodiment of the BIAcore^{®} assay the antigen is bound to a surface and binding of the antibody, i.e. its binding site(s), is measured by surface plasmon resonance (SPR). The affinity of the binding is defined by the terms kₐ (association constant: rate constant for the association to form a complex), k_{d} (dissociation constant; rate constant for the dissociation of the complex), and K_{D} (k_{d}/kₐ). Alternatively, the binding signal of a SPR sensorgram can be compared directly to the response signal of a reference, with respect to the resonance signal height and the dissociation behaviors.

The term "binding site" denotes any proteinaceous entity that shows binding specificity to a target. This can be, e.g., a receptor, a receptor ligand, an anticalin, an affibody, an antibody, etc. Thus, the term "binding site" as used herein denotes a polypeptide that can specifically bind to or can be specifically bound by a second polypeptide.

As used herein, the term "selection marker" denotes a gene that allows cells carrying the gene to be specifically selected for or against, in the presence of a corresponding selection agent. For example, but not by way of limitation, a selection marker can allow the host cell transformed with the selection marker gene to be positively selected for in the presence of the respective selection agent (selective cultivation conditions); a non-transformed host cell would not be capable of growing or surviving under the selective cultivation conditions. Selection markers can be positive, negative or bi-functional. Positive selection markers can allow selection for cells carrying the marker, whereas negative selection markers can allow cells carrying the marker to be selectively eliminated. A selection marker can confer resistance to a drug or compensate for a metabolic or catabolic defect in the host cell. In prokaryotic cells, amongst others, genes conferring resistance against ampicillin, tetracycline, kanamycin or chloramphenicol can be used. Resistance genes useful as selection markers in eukaryotic cells include, but are not limited to, genes for aminoglycoside phosphotransferase (APH) (e.g., hygromycin phosphotransferase (HYG), neomycin and G418 APH), dihydrofolate reductase (DHFR), thymidine kinase (TK), glutamine synthetase (GS), asparagine synthetase, tryptophan synthetase (indole), histidinol dehydrogenase (histidinol D), and genes encoding resistance to puromycin, blasticidin, bleomycin, phleomycin, chloramphenicol, Zeocin, and mycophenolic acid. Further marker genes are described in WO 92/08796 and WO 94/28143.

Beyond facilitating a selection in the presence of a corresponding selection agent, a selection marker can alternatively be a molecule normally not present in the cell, e.g., green fluorescent protein (GFP), enhanced GFP (eGFP), synthetic GFP, yellow fluorescent protein (YFP), enhanced YFP (eYFP), cyan fluorescent protein (CFP), mPlum, mCherry, tdTomato, mStrawberry, J-red, DsRed-monomer, mOrange, mKO, mCitrine, Venus, YPet, Emerald, CyPet, mCFPm, Cerulean, and T-Sapphire. Cells expressing such a molecule can be distinguished from cells not harboring this gene, e.g., by the detection or absence, respectively, of the fluorescence emitted by the encoded polypeptide.

As used herein, the term "operably linked" refers to a juxtaposition of two or more components, wherein the components are in a relationship permitting them to function in their intended manner. For example, a promoter and/or an enhancer is operably linked to a coding sequence if the promoter and/or enhancer acts to modulate the transcription of the coding sequence. In certain embodiments, DNA sequences that are "operably linked" are contiguous and adjacent on a single chromosome. In certain embodiments, e.g., when it is necessary to join two protein encoding regions, such as a secretory leader and a polypeptide, the sequences are contiguous, adjacent, and in the same reading frame. In certain embodiments, an operably linked promoter is located upstream of the coding sequence and can be adjacent to it. In certain embodiments, e.g., with respect to enhancer sequences modulating the expression of a coding sequence, the two components can be operably linked although not adjacent. An enhancer is operably linked to a coding sequence if the enhancer increases transcription of the coding sequence. Operably linked enhancers can be located upstream, within, or downstream of coding sequences and can be located at a considerable distance from the promoter of the coding sequence. Operable linkage can be accomplished by recombinant methods known in the art, e.g., using PCR methodology and/or by ligation at convenient restriction sites. If convenient restriction sites do not exist, then synthetic oligonucleotide adaptors or linkers can be used in accord with conventional practice. An internal ribosomal entry site (IRES) is operably linked to an open reading frame (ORF) if it allows initiation of translation of the ORF at an internal location in a 5' end-independent manner.

As used herein, the term "exogenous" indicates that a nucleotide sequence does not originate from a specific cell and is introduced into said cell by DNA delivery methods, e.g., by transfection, electroporation, or transformation methods. Thus, an exogenous nucleotide sequence is an artificial sequence wherein the artificiality can originate, e.g., from the combination of subsequences of different origin (e.g. a combination of a recombinase recognition sequence with an SV40 promoter and a coding sequence of green fluorescent protein is an artificial nucleic acid) or from the deletion of parts of a sequence (e.g. a sequence coding only the extracellular domain of a membrane-bound receptor or a cDNA) or the mutation of nucleobases. The term "endogenous" refers to a nucleotide sequence originating from a cell. An "exogenous" nucleotide sequence can have an "endogenous" counterpart that is identical in base compositions, but where the "exogenous" sequence is introduced into the cell, e.g., via recombinant DNA technology.

As used herein, the term "heterologous" indicates that a polypeptide does not originate from a specific cell and the respective encoding nucleic acid has been introduced into said cell by DNA delivery methods, e.g., by transfection, electroporation, or transformation methods. Thus, a heterologous polypeptide is a polypeptide that is artificial to the cell expressing it, whereby this is independent whether the polypeptide is a naturally occurring polypeptide originating from a different cell/organism or is a man-made polypeptide.

The term "sirtuin-1" denotes an enzyme that is part of signal transduction in mammals, i.e. the NAD-dependent deacetylase sirtuin-1. Sirtuin-1 is encoded by the SIRT-1 gene. Human sirtuin-1 has the UniProtKB entry Q96EB6 and is shown in SEQ ID NO: 17. Chinese hamster sirtuin-1 has the UniProtKB entry A0A3L7IF96 and is shown in SEQ ID NO: 18.

### II. ANTIBODIES

General information regarding the nucleotide sequences of human immunoglobulins light and heavy chains is given in: Kabat, E.A., et al., Sequences of Proteins of Immunological Interest, 5th ed., Public Health Service, National Institutes of Health, Bethesda, MD (1991).

As used herein, the amino acid positions of all constant regions and domains of the heavy and light chain are numbered according to the Kabat numbering system described in Kabat, et al., Sequences of Proteins of Immunological Interest, 5th ed., Public Health Service, National Institutes of Health, Bethesda, MD (1991) and is referred to as "numbering according to Kabat" herein. Specifically, the Kabat numbering system (see pages 647-660) of Kabat, et al., Sequences of Proteins of Immunological Interest, 5th ed., Public Health Service, National Institutes of Health, Bethesda, MD (1991) is used for the light chain constant domain CL of kappa and lambda isotype, and the Kabat EU index numbering system (see pages 661-723) of Kabat, et al., Sequences of Proteins of Immunological Interest, 5th ed., Public Health Service, National Institutes of Health, Bethesda, MD (1991) is used for the constant heavy chain domains (CH1, hinge, CH2 and CH3, which is herein further clarified by referring to "numbering according to Kabat EU index" in this case).

The term "antibody" herein is used in the broadest sense and encompasses various antibody structures, including but not limited to full length antibodies, monoclonal antibodies, multispecific antibodies (e.g., bispecific antibodies), and antibody-antibody fragment-fusions as well as combinations thereof.

The term "native antibody" denotes naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a heavy chain variable region (VH) followed by three heavy chain constant domains (CH1, CH2, and CH3), whereby between the first and the second heavy chain constant domain a hinge region is located. Similarly, from N- to C-terminus, each light chain has a light chain variable region (VL) followed by a light chain constant domain (CL). The light chain of an antibody may be assigned to one of two types, called kappa (κ) and lambda (λ), based on the amino acid sequence of its constant domain.

The term "full length antibody" denotes an antibody having a structure substantially similar to that of a native antibody. A full length antibody comprises two full length antibody light chains each comprising in N- to C-terminal direction a light chain variable region and a light chain constant domain, as well as two full length antibody heavy chains each comprising in N- to C-terminal direction a heavy chain variable region, a first heavy chain constant domain, a hinge region, a second heavy chain constant domain and a third heavy chain constant domain. In contrast to a native antibody, a full length antibody may comprise further immunoglobulin domains, such as e.g. one or more additional scFvs, or heavy or light chain Fab fragments, or scFabs conjugated to one or more of the termini of the different chains of the full length antibody, but only a single fragment to each terminus. These conjugates are also encompassed by the term full length antibody.

The term "antibody binding site" denotes a pair of a heavy chain variable domain and a light chain variable domain. To ensure proper binding to the antigen these variable domains are cognate variable domains, i.e. belong together. An antibody the binding site comprises at least three HVRs (e.g. in case of a VHH) or three-six HVRs (e.g. in case of a naturally occurring, i.e. conventional, antibody with a VH/VL pair). Generally, the amino acid residues of an antibody that are responsible for antigen binding are forming the binding site. These residues are normally contained in a pair of an antibody heavy chain variable domain and a corresponding antibody light chain variable domain. The antigen-binding site of an antibody comprises amino acid residues from the "hypervariable regions" or "HVRs". "Framework" or "FR" regions are those variable domain regions other than the hypervariable region residues as herein defined. Therefore, the light and heavy chain variable domains of an antibody comprise from N- to C-terminus the regions FR1, HVR1, FR2, HVR2, FR3, HVR3 and FR4. Especially, the HVR3 region of the heavy chain variable domain is the region, which contributes most to antigen binding and defines the binding specificity of an antibody. A "functional binding site" is capable of specifically binding to its target. The term "specifically binding to" denotes the binding of a binding site to its target in an in vitro assay, in one embodiment in a binding assay. Such binding assay can be any assay as long the binding event can be detected. For example, an assay in which the antibody is bound to a surface and binding of the antigen(s) to the antibody is measured by Surface Plasmon Resonance (SPR). Alternatively, a bridging ELISA can be used.

The term "hypervariable region" or "HVR", as used herein, refers to each of the regions of an antibody variable domain comprising the amino acid residue stretches which are hypervariable in sequence ("complementarity determining regions" or "CDRs") and/or form structurally defined loops ("hypervariable loops"), and/or contain the antigen-contacting residues ("antigen contacts"). Generally, antibodies comprise six HVRs; three in the heavy chain variable domain VH (H1, H2, H3), and three in the light chain variable domain VL (L1, L2, L3).

HVRs include
(a) hypervariable loops occurring at amino acid residues 26-32 (L1), 50-52 (L2), 91-96 (L3), 26-32 (H1), 53-55 (H2), and 96-101 (H3) (Chothia, C. and Lesk, A.M., J. Mol. Biol. 196 (1987) 901-917);
(b) CDRs occurring at amino acid residues 24-34 (L1), 50-56 (L2), 89-97 (L3), 31-35b (H1), 50-65 (H2), and 95-102 (H3) (Kabat, E.A. et al., Sequences of Proteins of Immunological Interest, 5th ed. Public Health Service, National Institutes of Health, Bethesda, MD (1991), NIH Publication 91-3242.);
(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and
(d) combinations of (a), (b), and/or (c), including amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Unless otherwise indicated, HVR residues and other residues in the variable domain (e.g., FR residues) are numbered herein according to Kabat et al., *supra.*

The "class" of an antibody refers to the type of constant domains or constant region, preferably the Fc-region, possessed by its heavy chains. There are five major classes of antibodies: IgA, IgD, IgE, IgG, and IgM, and several of these may be further divided into subclasses (isotypes), e.g., IgG1, IgG2, IgG3, IgG4, IgA1, and IgA2. The heavy chain constant domains that correspond to the different classes of immunoglobulins are called α, δ, ε, γ, and µ, respectively.

The term "heavy chain constant region" denotes the region of an immunoglobulin heavy chain that contains the constant domains, i.e. the CH1 domain, the hinge region, the CH2 domain and the CH3 domain. In one embodiment, a human IgG constant region extends from Ala118 to the carboxyl-terminus of the heavy chain (numbering according to Kabat EU index). However, the C-terminal lysine (Lys447) of the constant region may or may not be present (numbering according to Kabat EU index). The term "constant region" denotes a dimer comprising two heavy chain constant regions, which can be covalently linked to each other via the hinge region cysteine residues forming inter-chain disulfide bonds.

The term "heavy chain Fc-region" denotes the C-terminal region of an immunoglobulin heavy chain that contains at least a part of the hinge region (middle and lower hinge region), the CH2 domain and the CH3 domain. In one embodiment, a human IgG heavy chain Fc-region extends from Asp221, or from Cys226, or from Pro230, to the carboxyl-terminus of the heavy chain (numbering according to Kabat EU index). Thus, an Fc-region is smaller than a constant region but in the C-terminal part identical thereto. However, the C-terminal lysine (Lys447) of the heavy chain Fc-region may or may not be present (numbering according to Kabat EU index). The term "Fc-region" denotes a dimer comprising two heavy chain Fc-regions, which can be covalently linked to each other via the hinge region cysteine residues forming inter-chain disulfide bonds.

The constant region, more precisely the Fc-region, of an antibody (and the constant region likewise) is directly involved in complement activation, C1q binding, C3 activation and Fc receptor binding. While the influence of an antibody on the complement system is dependent on certain conditions, binding to C1q is caused by defined binding sites in the Fc-region. Such binding sites are known in the state of the art and described e.g. by Lukas, T.J., et al., J. Immunol. 127 (1981) 2555-2560; Brunhouse, R., and Cebra, J.J., Mol. Immunol. 16 (1979) 907-917; Burton, D.R., et al., Nature 288 (1980) 338-344; Thommesen, J.E., et al., Mol. Immunol. 37 (2000) 995-1004; Idusogie, E.E., et al., J. Immunol. 164 (2000) 4178-4184; Hezareh, M., et al., J. Virol. 75 (2001) 12161-12168; Morgan, A., et al., Immunology 86 (1995) 319-324; and EP 0 307 434. Such binding sites are e.g. L234, L235, D270, N297, E318, K320, K322, P331 and P329 (numbering according to EU index of Kabat). Antibodies of subclass IgG1, IgG2 and IgG3 usually show complement activation, C1q binding and C3 activation, whereas IgG4 do not activate the complement system, do not bind C1q and do not activate C3. An "Fc-region of an antibody" is a term well known to the skilled artisan and defined on the basis of papain cleavage of antibodies.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, monoclonal antibodies may be made by a variety of techniques, including but not limited to the hybridoma method, recombinant DNA methods, phage-display methods, and methods utilizing transgenic animals containing all or part of the human immunoglobulin loci.

The term "valent" as used within the current application denotes the presence of a specified number of binding sites in an antibody. As such, the terms "bivalent", "tetravalent", and "hexavalent" denote the presence of two binding site, four binding sites, and six binding sites, respectively, in an antibody.

A "monospecific antibody" denotes an antibody that has a single binding specificity, i.e. specifically binds to one antigen. Monospecific antibodies can be prepared as full-length antibodies or antibody fragments (e.g. F(ab')₂) or combinations thereof (e.g. full length antibody plus additional scFv or Fab fragments). A monospecific antibody does not need to be monovalent, i.e. a monospecific antibody may comprise more than one binding site specifically binding to the one antigen. A native antibody, for example, is monospecific but bivalent.

A "multispecific antibody" denotes an antibody that has binding specificities for at least two different epitopes on the same antigen or two different antigens. Multispecific antibodies can be prepared as full-length antibodies or antibody fragments (e.g. F(ab')₂ bispecific antibodies) or combinations thereof (e.g. full length antibody plus additional scFv or Fab fragments). A multispecific antibody is at least bivalent, i.e. comprises two antigen binding sites. Also a multispecific antibody is at least bispecific. Thus, a bivalent, bispecific antibody is the simplest form of a multispecific antibody. Engineered antibodies with two, three or more (e.g. four) functional antigen binding sites have also been reported (see, e.g., US 2002/0004587 A1).

In certain embodiments, the antibody is a multispecific antibody, e.g. at least a bispecific antibody. Multispecific antibodies are monoclonal antibodies that have binding specificities for at least two different antigens or epitopes. In certain embodiments, one of the binding specificities is for a first antigen and the other is for a different second antigen. In certain embodiments, multispecific antibodies may bind to two different epitopes of the same antigen. Multispecific antibodies may also be used to localize cytotoxic agents to cells, which express the antigen.

Multispecific antibodies can be prepared as full-length antibodies or antibody-antibody fragment-fusions.

Techniques for making multispecific antibodies include, but are not limited to, recombinant co-expression of two immunoglobulin heavy chain-light chain pairs having different specificities (see Milstein, C. and Cuello, A.C., Nature 305 (1983) 537-540, WO 93/08829, and Traunecker, A., et al., EMBO J. 10 (1991) 3655-3659), and "knob-in-hole" engineering (see, e.g., US 5,731,168). Multi-specific antibodies may also be made by engineering electrostatic steering effects for making antibody Fc-heterodimeric molecules (WO 2009/089004); cross-linking two or more antibodies or fragments (see, e.g., US 4,676,980, and Brennan, M., et al., Science 229 (1985) 81-83); using leucine zippers to produce bi-specific antibodies (see, e.g., Kostelny, S.A., et al., J. Immunol. 148 (1992) 1547-1553); using the common light chain technology for circumventing the light chain mis-pairing problem (see, e.g., WO 98/50431); using specific technology for making bispecific antibody fragments (see, e.g., Holliger, P., et al., Proc. Natl. Acad. Sci. USA 90 (1993) 6444-6448); and preparing trispecific antibodies as described, e.g., in Tutt, A., et al., J. Immunol. 147 (1991) 60-69).

Engineered antibodies with three or more antigen binding sites, including for example, "Octopus antibodies", or DVD-Ig are also included herein (see, e.g., WO 2001/77342 and WO 2008/024715). Other examples of multispecific antibodies with three or more antigen binding sites can be found in WO 2010/115589, WO 2010/112193, WO 2010/136172, WO 2010/145792, and WO 2013/026831. The bispecific antibody or antigen binding fragment thereof also includes a "Dual Acting Fab" or "DAF" (see, e.g., US 2008/0069820 and WO 2015/095539).

Multi-specific antibodies may also be provided in an asymmetric form with a domain crossover in one or more binding arms of the same antigen specificity, i.e. by exchanging the VH/VL domains (see e.g., WO 2009/080252 and WO 2015/150447), the CH1/CL domains (see e.g., WO 2009/080253) or the complete Fab arms (see e.g., WO 2009/080251, WO 2016/016299, also see Schaefer et al, PNAS, 108 (2011) 1187-1191, and Klein at al., MAbs 8 (2016) 1010-20). In one aspect, the multispecific antibody comprises a Cross-Fab fragment. The term "Cross-Fab fragment" or "xFab fragment" or "crossover Fab fragment" refers to a Fab fragment, wherein either the variable regions or the constant regions of the heavy and light chain are exchanged. A Cross-Fab fragment comprises a polypeptide chain composed of the light chain variable region (VL) and the heavy chain constant region 1 (CH1), and a polypeptide chain composed of the heavy chain variable region (VH) and the light chain constant region (CL). Asymmetrical Fab arms can also be engineered by introducing charged or non-charged amino acid mutations into domain interfaces to direct correct Fab pairing. See e.g., WO 2016/172485.

The antibody or fragment can also be a multispecific antibody as described in WO 2009/080254, WO 2010/112193, WO 2010/115589, WO 2010/136172, WO 2010/145792, or WO 2010/145793.

The antibody or fragment thereof may also be a multispecific antibody as disclosed in WO 2012/163520.

Various further molecular formats for multispecific antibodies are known in the art and are included herein (see e.g., Spiess et al., Mol. Immunol. 67 (2015) 95-106).

Bispecific antibodies are generally antibody molecules that specifically bind to two different, non-overlapping epitopes on the same antigen or to two epitopes on different antigens.

Complex (multispecific) antibodies are
- full-length antibody with domain exchange:
   a multispecific IgG antibody comprising a first Fab fragment and a second Fab fragment, wherein in the first Fab fragment
      a) only the CH1 and CL domains are replaced by each other (i.e. the light chain of the first Fab fragment comprises a VL and a CH1 domain and the heavy chain of the first Fab fragment comprises a VH and a CL domain);
      b) only the VH and VL domains are replaced by each other (i.e. the light chain of the first Fab fragment comprises a VH and a CL domain and the heavy chain of the first Fab fragment comprises a VL and a CH1 domain); or
      c) the CH1 and CL domains are replaced by each other and the VH and VL domains are replaced by each other (i.e. the light chain of the first Fab fragment comprises a VH and a CH1 domain and the heavy chain of the first Fab fragment comprises a VL and a CL domain); and
   wherein the second Fab fragment comprises a light chain comprising a VL and a CL domain, and a heavy chain comprising a VH and a CH1 domain;
   the full-length antibody with domain exchange may comprises a first heavy chain including a CH3 domain and a second heavy chain including a CH3 domain, wherein both CH3 domains are engineered in a complementary manner by respective amino acid substitutions, in order to support heterodimerization of the first heavy chain and the modified second heavy chain, e.g. as disclosed in WO 96/27011, WO 98/050431, EP 1870459, WO 2007/110205, WO 2007/147901, WO 2009/089004, WO 2010/129304, WO 2011/90754, WO 2011/143545, WO 2012/058768, WO 2013/157954, or WO 2013/096291 (incorporated herein by reference);
- full-length antibody with domain exchange and additional heavy chain C-terminal binding site:
   a multispecific IgG antibody comprising
      a) one full length antibody comprising two pairs each of a full length antibody light chain and a full length antibody heavy chain, wherein the binding sites formed by each of the pairs of the full length heavy chain and the full length light chain specifically bind to a first antigen, and
      b) one additional Fab fragment, wherein the additional Fab fragment is fused to the C-terminus of one heavy chain of the full length antibody, wherein the binding site of the additional Fab fragment specifically binds to a second antigen,
   wherein the additional Fab fragment specifically binding to the second antigen i) comprises a domain crossover such that a) the light chain variable domain (VL) and the heavy chain variable domain (VH) are replaced by each other, or b) the light chain constant domain (CL) and the heavy chain constant domain (CH1) are replaced by each other, or ii) is a single chain Fab fragment;
- the one-armed single chain format (= one-armed single chain antibody): antibody comprising a first binding site that specifically binds to a first epitope or antigen and a second binding site that specifically binds to a second epitope or antigen, whereby the individual chains are as follows
   - light chain (variable light chain domain + light chain kappa constant domain)
   - combined light/heavy chain (variable light chain domain + light chain constant domain + peptidic linker + variable heavy chain domain + CH1 + Hinge + CH2 + CH3 with knob mutation)
   - heavy chain (variable heavy chain domain + CH1 + Hinge + CH2 + CH3 with hole mutation);
- the two-armed single chain format (= two-armed single chain antibody): antibody comprising a first binding site that specifically binds to a first epitope or antigen and a second binding site that specifically binds to a second epitope or antigen, whereby the individual chains are as follows
   - combined light/heavy chain 1 (variable light chain domain + light chain constant domain + peptidic linker + variable heavy chain domain + CH1 + Hinge + CH2 + CH3 with hole mutation)
   - combined light/heavy chain 2 (variable light chain domain + light chain constant domain + peptidic linker + variable heavy chain domain + CH1 + Hinge + CH2 + CH3 with knob mutation);
- the common light chain bispecific format (= common light chain bispecific antibody):
   antibody comprising a first binding site that specifically binds to a first epitope or antigen and a second binding site that specifically binds to a second epitope or antigen, whereby the individual chains are as follows
   - light chain (variable light chain domain + light chain constant domain)
   - heavy chain 1 (variable heavy chain domain + CH1 + Hinge + CH2 + CH3 with hole mutation)
   - heavy chain 2 (variable heavy chain domain + CH1 + Hinge + CH2 + CH3 with knob mutation);
- the T-cell bispecific format:
   a full-length antibody with additional heavy chain N-terminal binding site with domain exchange comprising
   - a first and a second Fab fragment, wherein each binding site of the first and the second Fab fragment specifically bind to a first antigen,
   - a third Fab fragment, wherein the binding site of the third Fab fragment specifically binds to a second antigen, and wherein the third Fab fragment comprises a domain crossover such that the variable light chain domain (VL) and the variable heavy chain domain (VH) are replaced by each other, and
   - an Fc-region comprising a first Fc-region polypeptide and a second Fc-region polypeptide,
      wherein the first and the second Fab fragment each comprise a heavy chain fragment and a full length light chain,
      wherein the C-terminus of the heavy chain fragment of the first Fab fragment is fused to the N-terminus of the first Fc-region polypeptide,
      wherein the C-terminus of the heavy chain fragment of the second Fab fragment is fused to the N-terminus of the variable light chain domain of the third Fab fragment and the C-terminus of the CH1 domain of the third Fab fragment is fused to the N-terminus of the second Fc-region polypeptide.

The "knobs into holes" dimerization modules and their use in antibody engineering are described in Carter P.; Ridgway J.B.B.; Presta L.G.: Immunotechnology, Volume 2, Number 1, February 1996, pp. 73-73(1).

The CH3 domains in the heavy chains of an antibody can be altered by the "knob-into-holes" technology, which is described in detail with several examples in e.g. WO 96/027011, Ridgway, J.B., et al., Protein Eng. 9 (1996) 617-621; and Merchant, A.M., et al., Nat. Biotechnol. 16 (1998) 677-681. In this method the interaction surfaces of the two CH3 domains are altered to increase the heterodimerization of these two CH3 domains and thereby of the polypeptide comprising them. Each of the two CH3 domains (of the two heavy chains) can be the "knob", while the other is the "hole". The introduction of a disulfide bridge further stabilizes the heterodimers (Merchant, A.M., et al., Nature Biotech. 16 (1998) 677-681; Atwell, S., et al., J. Mol. Biol. 270 (1997) 26-35) and increases the yield.

The mutation T366W in the CH3 domain (of an antibody heavy chain) is denoted as "knob-mutation" or "mutation knob" and the mutations T366S, L368A, Y407V in the CH3 domain (of an antibody heavy chain) are denoted as "hole-mutations" or "mutations hole" (numbering according to Kabat EU index). An additional inter-chain disulfide bridge between the CH3 domains can also be used (Merchant, A.M., et al., Nature Biotech. 16 (1998) 677-681) e.g. by introducing a S354C mutation into the CH3 domain of the heavy chain with the "knob-mutation" (denotes as "knob-cys-mutations" or "mutations knob-cys") and by introducing a Y349C mutation into the CH3 domain of the heavy chain with the "hole-mutations" (denotes as "hole-cys-mutations" or "mutations hole-cys") (numbering according to Kabat EU index).

The term "domain crossover" as used herein denotes that in a pair of an antibody heavy chain VH-CH1 fragment and its corresponding cognate antibody light chain, i.e. in an antibody Fab (fragment antigen binding), the domain sequence deviates from the sequence in a native antibody in that at least one heavy chain domain is substituted by its corresponding light chain domain and vice versa. There are three general types of domain crossovers, (i) the crossover of the CH1 and the CL domains, which leads by the domain crossover in the light chain to a VL-CH1 domain sequence and by the domain crossover in the heavy chain fragment to a VH-CL domain sequence (or a full length antibody heavy chain with a VH-CL-hinge-CH2-CH3 domain sequence), (ii) the domain crossover of the VH and the VL domains, which leads by the domain crossover in the light chain to a VH-CL domain sequence and by the domain crossover in the heavy chain fragment to a VL-CH1 domain sequence, and (iii) the domain crossover of the complete light chain (VL-CL) and the complete VH-CH1 heavy chain fragment ("Fab crossover"), which leads to by domain crossover to a light chain with a VH-CH1 domain sequence and by domain crossover to a heavy chain fragment with a VL-CL domain sequence (all aforementioned domain sequences are indicated in N-terminal to C-terminal direction).

As used herein the term "replaced by each other" with respect to corresponding heavy and light chain domains refers to the aforementioned domain crossovers. As such, when CH1 and CL domains are "replaced by each other" it is referred to the domain crossover mentioned under item (i) and the resulting heavy and light chain domain sequence. Accordingly, when VH and VL are "replaced by each other" it is referred to the domain crossover mentioned under item (ii); and when the CH1 and CL domains are "replaced by each other" and the VH and VL domains are "replaced by each other" it is referred to the domain crossover mentioned under item (iii). Bispecific antibodies including domain crossovers are reported, e.g. in WO 2009/080251, WO 2009/080252, WO 2009/080253, WO 2009/080254 and Schaefer, W., et al, Proc. Natl. Acad. Sci USA 108 (2011) 11187-11192. Such antibodies are generally termed CrossMab.

Multispecific antibodies also comprise in one embodiment at least one Fab fragment including either a domain crossover of the CH1 and the CL domains as mentioned under item (i) above, or a domain crossover of the VH and the VL domains as mentioned under item (ii) above, or a domain crossover of the VH-CH1 and the VL-VL domains as mentioned under item (iii) above. In case of multispecific antibodies with domain crossover, the Fabs specifically binding to the same antigen(s) are constructed to be of the same domain sequence. Hence, in case more than one Fab with a domain crossover is contained in the multispecific antibody, said Fab(s) specifically bind to the same antigen.

A "humanized" antibody refers to an antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., the CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "recombinant antibody", as used herein, denotes all antibodies (chimeric, humanized and human) that are prepared, expressed, created or isolated by recombinant means, such as recombinant cells. This includes antibodies isolated from recombinant cells such as NS0, HEK, BHK, amniocyte or CHO cells.

As used herein, the term "antibody fragment" refers to a molecule other than an intact antibody that comprises a portion of an intact antibody that binds the antigen to which the intact antibody binds, i.e. it is a functional fragment. Examples of antibody fragments include but are not limited to Fv; Fab; Fab'; Fab'-SH; F(ab')2; bispecific Fab; diabodies; linear antibodies; single-chain antibody molecules (e.g., scFv or scFab).

### III. RECOMBINANT METHODS AND COMPOSITIONS

Antibodies may be produced using recombinant methods and compositions, e.g., as described in US 4,816,567. For these methods one or more isolated nucleic acid(s) encoding an antibody are provided.

In one aspect, a method of making an antibody is provided, wherein the method comprises culturing a host cell comprising nucleic acid(s) encoding the antibody, as provided above, under conditions suitable for expression of the antibody, and optionally recovering the antibody from the host cell (or host cell culture medium).

For recombinant production of an antibody, nucleic acids encoding the antibody, e.g., as described above, are isolated and inserted into one or more vectors for further cloning and/or expression in a host cell. Such nucleic acids may be readily isolated and sequenced using conventional procedures (e.g., by using oligonucleotide probes that are capable of binding specifically to genes encoding the heavy and light chains of the antibody) or produced by recombinant methods or obtained by chemical synthesis.

Generally, for the recombinant large scale production of a polypeptide of interest, such as e.g. a therapeutic antibody, a cell stably expressing and secreting said polypeptide is required. This cell is termed "recombinant cell" or "recombinant production cell" and the process used for generating such a cell is termed "cell line development". In the first step of the cell line development process, a suitable host cell, such as e.g. a CHO cell, is transfected with a nucleic acid sequence suitable for expression of said polypeptide of interest. In a second step a cell stably expressing the polypeptide of interest is selected based on the co-expression of a selection marker, which had been co-transfected with the nucleic acid encoding the polypeptide of interest.

A nucleic acid encoding a polypeptide, i.e. the coding sequence, is called a structural gene. Such a structural gene is simple information and additional regulatory elements are required for expression thereof. Therefore, normally a structural gene is integrated in a so called expression cassette. The minimal regulatory elements needed for an expression cassette to be functional in a mammalian cell are a promoter functional in said mammalian cell, which is located upstream, i.e. 5', to the structural gene, and a polyadenylation signal sequence functional in said mammalian cell, which is located downstream, i.e. 3', to the structural gene. The promoter, the structural gene and the polyadenylation signal sequence are arranged in an operably linked form.

In case the polypeptide of interest is a heteromultimeric polypeptide that is composed of different (monomeric) polypeptides, such as e.g. an antibody or a complex antibody format, not only a single expression cassette is required but a multitude of expression cassettes differing in the contained structural gene, i.e. at least one expression cassette for each of the different (monomeric) polypeptides of the heteromultimeric polypeptide. For example, a full length antibody is a heteromultimeric polypeptide comprising two copies of a light chain as well as two copies of a heavy chain. Thus, a full length antibody is composed of two different polypeptides. Therefore, two expression cassettes are required for the expression of a full length antibody, one for the light chain and one for the heavy chain. If, for example, the full length antibody is a bispecific antibody, i.e. the antibody comprises two different binding sites specifically binding to two different antigens, the two light chains as well as the two heavy chains are also different from each other. Thus, such a bispecific, full length antibody is composed of four different polypeptides and therefore, four expression cassettes are required.

The expression cassette(s) for the polypeptide of interest is(are) in turn integrated into one or more so called "expression vector(s)". An "expression vector" is a nucleic acid providing all required elements for the amplification of said vector in bacterial cells as well as the expression of the comprised structural gene(s) in a mammalian cell. Typically, an expression vector comprises a prokaryotic plasmid propagation unit, e.g. for E.coli, comprising an origin of replication, and a prokaryotic selection marker, as well as a eukaryotic selection marker, and the expression cassettes required for the expression of the structural gene(s) of interest. An "expression vector" is a transport vehicle for the introduction of expression cassettes into a mammalian cell.

As outlined in the previous paragraphs, the more complex the polypeptide to be expressed is the higher also the number of required different expression cassettes is. Inherently with the number of expression cassettes also the size of the nucleic acid to be integrated into the genome of the host cell increases. Concomitantly also the size of the expression vector increases. But there is a practical upper limit to the size of a vector in the range of about 15 kbps above which handling and processing efficiency profoundly drops. This issue can be addressed by using two or more expression vectors. Thereby the expression cassettes can be split between different expression vectors each comprising only some of the expression cassettes resulting in a size reduction.

Cell line development (CLD) for the generation of recombinant cell expressing a heterologous polypeptide, such as e.g. a multispecific antibody, employs either random integration (RI) or targeted integration (TI) of the nucleic acid(s) comprising the respective expression cassettes required for the expression and production of the heterologous polypeptide of interest.

Using RI, in general, several vectors or fragments thereof integrate into the cell's genome at the same or different loci.

Using TI, in general, a single copy of the transgene comprising the different expression cassettes is integrated at a predetermined "hot-spot" in the host cell's genome.

Suitable host cells for the expression of an (glycosylated) antibody are generally derived from multicellular organisms such as e.g. vertebrates.

### IV. HOST CELLS

Any mammalian cell line that is adapted to grow in suspension can be used in the method according to the current invention. Also independent from the integration method, i.e. for RI as well as TI, any mammalian host cell can be used.

Examples of useful mammalian host cell lines are human amniocyte cells (e.g. CAP-T cells as described in Woelfel, J. et al., BMC Proc. 5 (2011) P133); monkey kidney CV1 line transformed by SV40 (COS-7); human embryonic kidney line (HEK293 or HEK293T cells as described, e.g., in Graham, F.L. et al., J. Gen Virol. 36 (1977) 59-74); baby hamster kidney cells (BHK); mouse sertoli cells (TM4 cells as described, e.g., in Mather, J.P., Biol. Reprod. 23 (1980) 243-252); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells (as described, e.g., in Mather, J.P. et al., Annals N.Y. Acad. Sci. 383 (1982) 44-68); MRC 5 cells; and FS4 cells. Other useful mammalian host cell lines include Chinese hamster ovary (CHO) cells, including DHFR-CHO cells (Urlaub, G. et al., Proc. Natl. Acad. Sci. USA 77 (1980) 4216-4220); and myeloma cell lines such as Y0, NS0 and Sp2/0. For a review of certain mammalian host cell lines suitable for antibody production, see, e.g., Yazaki, P. and Wu, A.M., Methods in Molecular Biology, Vol. 248, Lo, B.K.C. (ed.), Humana Press, Totowa, NJ (2004), pp. 255-268.

In one embodiment, the mammalian host cell is, e.g., a Chinese Hamster Ovary (CHO) cell (e.g. CHO K1, CHO DG44, etc.), a Human Embryonic Kidney (HEK) cell, a lymphoid cell (e.g., Y0, NS0, Sp20 cell), or a human amniocyte cells (e.g. CAP-T, etc.). In one preferred embodiment the mammalian host cell is a CHO cell.

Targeted integration allows for exogenous nucleotide sequences to be integrated into a pre-determined site of a mammalian cell's genome. In certain embodiments, the targeted integration is mediated by a recombinase that recognizes one or more recombination recognition sequences (RRSs), which are present in the genome and in the exogenous nucleotide sequence to be integrated. In certain embodiments, the targeted integration is mediated by homologous recombination.

A "recombination recognition sequence" (RRS) is a nucleotide sequence recognized by a recombinase and is necessary and sufficient for recombinase-mediated recombination events. A RRS can be used to define the position where a recombination event will occur in a nucleotide sequence.

In certain embodiments, a RRS can be recognized by a Cre recombinase. In certain embodiments, a RRS can be recognized by a FLP recombinase. In certain embodiments, a RRS can be recognized by a Bxb1 integrase. In certain embodiments, a RRS can be recognized by a φC31 integrase.

In certain embodiments when the RRS is a LoxP site, the cell requires the Cre recombinase to perform the recombination. In certain embodiments when the RRS is a FRT site, the cell requires the FLP recombinase to perform the recombination. In certain embodiments when the RRS is a Bxb1 attP or a Bxb1 attB site, the cell requires the Bxb1 integrase to perform the recombination. In certain embodiments when the RRS is a φC31 attP or a φC31attB site, the cell requires the φC31 integrase to perform the recombination. The recombinases can be introduced into a cell using an expression vector comprising coding sequences of the enzymes or as protein or a mRNA.

With respect to TI, any known or future mammalian host cell suitable for TI comprising a landing site as described herein integrated at a single site within a locus of the genome can be used in the current invention. Such a cell is denoted as mammalian TI host cell. In certain embodiments, the mammalian TI host cell is a hamster cell, a human cell, a rat cell, or a mouse cell comprising a landing site as described herein. In one preferred embodiment the mammalian TI host cell is a CHO cell. In certain embodiments, the mammalian TI host cell is a Chinese hamster ovary (CHO) cell, a CHO K1 cell, a CHO K1SV cell, a CHO DG44 cell, a CHO DUKXB-11 cell, a CHO K1S cell, or a CHO K1M cell comprising a landing site as described herein integrated at a single site within a locus of the genome.

In certain embodiments, a mammalian TI host cell comprises an integrated landing site, wherein the landing site comprises one or more recombination recognition sequence (RRS). The RRS can be recognized by a recombinase, for example, a Cre recombinase, an FLP recombinase, a Bxb1 integrase, or a φC31 integrase. The RRS can be selected independently of each other from the group consisting of a LoxP sequence, a LoxP L3 sequence, a LoxP 2L sequence, a LoxFas sequence, a Lox511 sequence, a Lox2272 sequence, a Lox2372 sequence, a Lox5171 sequence, a Loxm2 sequence, a Lox71 sequence, a Lox66 sequence, a FRT sequence, a Bxb1 attP sequence, a Bxb1 attB sequence, a φC31 attP sequence, and a φC31 attB sequence. If multiple RRSs have to be present, the selection of each of the sequences is dependent on the other insofar as non-identical RRSs are chosen.

In certain embodiments, the landing site comprises one or more recombination recognition sequence (RRS), wherein the RRS can be recognized by a recombinase. In certain embodiments, the integrated landing site comprises at least two RRSs. In certain embodiments, an integrated landing site comprises three RRSs, wherein the third RRS is located between the first and the second RRS. In certain preferred embodiments, all three RRSs are different. In certain embodiments, the landing site comprises a first, a second and a third RRS, and at least one selection marker located between the first and the second RRS, and the third RRS is different from the first and/or the second RRS. In certain embodiments, the landing site further comprises a second selection marker, and the first and the second selection markers are different. In certain embodiments, the landing site further comprises a third selection marker and an internal ribosome entry site (IRES), wherein the IRES is operably linked to the third selection marker. The third selection marker can be different from the first or the second selection marker.

Although the invention is exemplified with a CHO cell hereafter, this is presented solely to exemplify the invention but shall not be construed in any way as limitation. The true scope of the invention is set forth in the claims.

An exemplary mammalian TI host cell that is suitable for use in a method according to the current invention is a CHO cell harboring a landing site integrated at a single site within a locus of its genome wherein the landing site comprises three heterospecific loxP sites for Cre recombinase mediated DNA recombination.

In this example, the heterospecific loxP sites are L3, LoxFas and 2L (see e.g. Lanza et al., Biotechnol. J. 7 (2012) 898-908; Wong et al., Nucleic Acids Res. 33 (2005) e147), whereby L3 and 2L flank the landing site at the 5'-end and 3'-end, respectively, and LoxFas is located between the L3 and 2L sites. The landing site further contains a bicistronic unit linking the expression of a selection marker via an IRES to the expression of the fluorescent GFP protein allowing to stabilize the landing site by positive selection as well as to select for the absence of the site after transfection and Cre-recombination (negative selection). Green fluorescence protein (GFP) serves for monitoring the RMCE reaction.

Such a configuration of the landing site as outlined in the previous paragraph allows for the simultaneous integration of two vectors, e.g. of a so called front vector harboring an L3 and a LoxFas site and a back vector harboring a LoxFas and an 2L site. The functional elements of a selection marker gene different from that present in the landing site can be distributed between both vectors: promoter and start codon can be located on the front vector whereas coding region and poly A signal are located on the back vector. Only correct recombinase-mediated integration of said nucleic acids from both vectors induces resistance against the respective selection agent.

Generally, a mammalian TI host cell is a mammalian cell comprising a landing site integrated at a single site within a locus of the genome of the mammalian cell, wherein the landing site comprises a first and a second recombination recognition sequence flanking at least one first selection marker, and a third recombination recognition sequence located between the first and the second recombination recognition sequence, and all the recombination recognition sequences are different.

The selection marker(s) can be selected from the group consisting of an aminoglycoside phosphotransferase (APH) (e.g., hygromycin phosphotransferase (HYG), neomycin and G418 APH), dihydrofolate reductase (DHFR), thymidine kinase (TK), glutamine synthetase (GS), asparagine synthetase, tryptophan synthetase (indole), histidinol dehydrogenase (histidinol D), and genes encoding resistance to puromycin, blasticidin, bleomycin, phleomycin, chloramphenicol, Zeocin, and mycophenolic acid. The selection marker(s) can also be a fluorescent protein selected from the group consisting of green fluorescent protein (GFP), enhanced GFP (eGFP), a synthetic GFP, yellow fluorescent protein (YFP), enhanced YFP (eYFP), cyan fluorescent protein (CFP), mPlum, mCherry, tdTomato, mStrawberry, J-red, DsRed-monomer, mOrange, mKO, mCitrine, Venus, YPet, Emerald6, CyPet, mCFPm, Cerulean, and T-Sapphire.

An exogenous nucleotide sequence is a nucleotide sequence that does not originate from a specific cell but can be introduced into said cell by DNA delivery methods, such as, e.g., by transfection, electroporation, or transformation methods. In certain embodiments, a mammalian TI host cell comprises at least one landing site integrated at one or more integration sites in the mammalian cell's genome. In certain embodiments, the landing site is integrated at one or more integration sites within a specific a locus of the genome of the mammalian cell.

In certain embodiments, the integrated landing site comprises at least one selection marker. In certain embodiments, the integrated landing site comprises a first, a second and a third RRS, and at least one selection marker. In certain embodiments, a selection marker is located between the first and the second RRS. In certain embodiments, two RRSs flank at least one selection marker, i.e., a first RRS is located 5' (upstream) and a second RRS is located 3' (downstream) of the selection marker. In certain embodiments, a first RRS is adjacent to the 5'-end of the selection marker and a second RRS is adjacent to the 3'-end of the selection marker. In certain embodiments, the landing site comprises a first, second, and third RRS, and at least one selection marker located between the first and the third RRS.

In certain embodiments, a selection marker is located between a first and a second RRS and the two flanking RRSs are different. In certain preferred embodiments, the first flanking RRS is a LoxP L3 sequence and the second flanking RRS is a LoxP 2L sequence. In certain embodiments, a LoxP L3 sequenced is located 5' of the selection marker and a LoxP 2L sequence is located 3' of the selection marker. In certain embodiments, the first flanking RRS is a wild-type FRT sequence and the second flanking RRS is a mutant FRT sequence. In certain embodiments, the first flanking RRS is a Bxb1 attP sequence and the second flanking RRS is a Bxb1 attB sequence. In certain embodiments, the first flanking RRS is a φC31 attP sequence and the second flanking RRS is a φC31 attB sequence. In certain embodiments, the two RRSs are positioned in the same orientation. In certain embodiments, the two RRSs are both in the forward or reverse orientation. In certain embodiments, the two RRSs are positioned in opposite orientation.

In certain embodiments, the integrated landing site comprises a first and a second selection marker, which are flanked by two RRSs, wherein the first selection marker is different from the second selection marker. In certain embodiments, the two selection markers are both independently of each other selected from the group consisting of a glutamine synthetase selection marker, a thymidine kinase selection marker, a HYG selection marker, and a puromycin resistance selection marker. In certain embodiments, the integrated landing site comprises a thymidine kinase selection marker and a HYG selection marker. In certain embodiments, the first selection maker is selected from the group consisting of an aminoglycoside phosphotransferase (APH) (e.g., hygromycin phosphotransferase (HYG), neomycin and G418 APH), dihydrofolate reductase (DHFR), thymidine kinase (TK), glutamine synthetase (GS), asparagine synthetase, tryptophan synthetase (indole), histidinol dehydrogenase (histidinol D), and genes encoding resistance to puromycin, blasticidin, bleomycin, phleomycin, chloramphenicol, Zeocin, and mycophenolic acid, and the second selection maker is selected from the group consisting of a GFP, an eGFP, a synthetic GFP, a YFP, an eYFP, a CFP, an mPlum, an mCherry, a tdTomato, an mStrawberry, a J-red, a DsRed-monomer, an mOrange, an mKO, an mCitrine, a Venus, a YPet, an Emerald, a CyPet, an mCFPm, a Cerulean, and a T-Sapphire fluorescent protein. In certain embodiments, the first selection marker is a glutamine synthetase selection marker and the second selection marker is a GFP fluorescent protein. In certain embodiments, the two RRSs flanking both selection markers are different.

In certain embodiments, the selection marker is operably linked to a promoter sequence. In certain embodiments, the selection marker is operably linked to an SV40 promoter. In certain embodiments, the selection marker is operably linked to a human Cytomegalovirus (CMV) promoter.

### V. TARGETED INTEGRATION

One method for the generation of a recombinant mammalian cell according to the current invention is targeted integration (TI).

In targeted integration site-specific recombination is employed for the introduction of an exogenous nucleic acid into a specific locus in the genome of a mammalian TI host cell. This is an enzymatic process wherein a sequence at the site of integration in the genome is exchanged for the exogenous nucleic acid. One system used to effect such nucleic acid exchanges is the Cre-lox system. The enzyme catalyzing the exchange is the Cre recombinase. The sequence to be exchanged is defined by the position of two lox(P)-sites in the genome as well as in the exogenous nucleic acid. These lox(P)-sites are recognized by the Cre recombinase. Nothing more is required, i.e. no ATP etc. Originally the Cre-lox system has been found in bacteriophage P1.

The Cre-lox system operates in different cell types, like mammals, plants, bacteria and yeast.

In one embodiment the exogenous nucleic acid encoding the heterologous polypeptide has been integrated into the mammalian TI host cell by single or double recombinase mediated cassette exchange (RMCE). Thereby a recombinant mammalian cell, such as a recombinant CHO cell, is obtained, in which a defined and specific expression cassette sequence has been integrated into the genome at a single locus, which in turn results in the efficient expression and production of the heterologous polypeptide.

The Cre-LoxP site-specific recombination system has been widely used in many biological experimental systems. Cre recombinase is a 38-kDa site-specific DNA recombinase that recognizes 34 bp LoxP sequences. Cre recombinase is derived from bacteriophage P1 and belongs to the tyrosine family site-specific recombinase. Cre recombinase can mediate both intra and intermolecular recombination between LoxP sequences. The LoxP sequence is composed of an 8 bp non-palindromic core region flanked by two 13 bp inverted repeats. Cre recombinase binds to the 13 bp repeat thereby mediating recombination within the 8 bp core region. Cre-LoxP-mediated recombination occurs at a high efficiency and does not require any other host factors. If two LoxP sequences are placed in the same orientation on the same nucleotide sequence, Cre recombinase-mediated recombination will excise DNA sequences located between the two LoxP sequences as a covalently closed circle. If two LoxP sequences are placed in an inverted position on the same nucleotide sequence, Cre recombinase-mediated recombination will invert the orientation of the DNA sequences located between the two sequences. If two LoxP sequences are on two different DNA molecules and if one DNA molecule is circular, Cre recombinase-mediated recombination will result in integration of the circular DNA sequence.

The term "matching RRSs" indicates that a recombination occurs between two RRSs. In certain embodiments, the two matching RRSs are the same. In certain embodiments, both RRSs are wild-type LoxP sequences. In certain embodiments, both RRSs are mutant LoxP sequences. In certain embodiments, both RRSs are wild-type FRT sequences. In certain embodiments, both RRSs are mutant FRT sequences. In certain embodiments, the two matching RRSs are different sequences but can be recognized by the same recombinase. In certain embodiments, the first matching RRS is a Bxb1 attP sequence and the second matching RRS is a Bxb1 attB sequence. In certain embodiments, the first matching RRS is a φC31 attB sequence and the second matching RRS is a φC31 attB sequence.

A "two-plasmid RMCE" strategy or "double RMCE" is employed in the method according to the current invention when using a two vector combination. For example, but not by way of limitation, an integrated landing site could comprise three RRSs, e.g., an arrangement where the third RRS ("RRS3") is present between the first RRS ("RRS1") and the second RRS ("RRS2"), while a first vector comprises two RRSs matching the first and the third RRS on the integrated exogenous nucleotide sequence, and a second vector comprises two RRSs matching the third and the second RRS on the integrated exogenous nucleotide sequence.

The two-plasmid RMCE strategy involves using three RRS sites to carry out two independent RMCEs simultaneously. Therefore, a landing site in the mammalian TI host cell using the two-plasmid RMCE strategy includes a third RRS site (RRS3) that has no cross activity with either the first RRS site (RRS1) or the second RRS site (RRS2). The two plasmids to be targeted require the same flanking RRS sites for efficient targeting, one plasmid (front) flanked by RRS1 and RRS3 and the other (back) by RRS3 and RRS2. Also two selection markers are needed in the two-plasmid RMCE. One selection marker expression cassette was split into two parts. The front plasmid would contain the promoter followed by a start codon and the RRS3 sequence. The back plasmid would have the RRS3 sequence fused to the N-terminus of the selection marker coding region, minus the start-codon (ATG). Additional nucleotides may need to be inserted between the RRS3 site and the selection marker sequence to ensure in frame translation for the fusion protein, i.e. operable linkage. Only when both plasmids are correctly inserted the full expression cassette of the selection marker will be assembled and, thus, rendering cells resistance to the respective selection agent.

Two-plasmid RMCE involves double recombination cross-over events, catalyzed by a recombinase, between the two heterospecific RRSs within the target genomic locus and the donor DNA molecule. Two-plasmid RMCE is designed to introduce a copy of the DNA sequences from the front- and back-vector in combination into the pre-determined locus of a mammalian TI host cell's genome. RMCE can be implemented such that prokaryotic vector sequences are not introduced into the mammalian TI host cell's genome, thus, reducing and/or preventing unwanted triggering of host immune or defense mechanisms. The RMCE procedure can be repeated with multiple DNA sequences.

In certain embodiments, targeted integration is achieved by two RMCEs, wherein two different DNA sequences, each comprising at least one expression cassette encoding a part of a heteromultimeric polypeptide and/or at least one selection marker or part thereof flanked by two heterospecific RRSs, are both integrated into a pre-determined site of the genome of a RRSs matching mammalian TI host cell. In certain embodiments, targeted integration is achieved by multiple RMCEs, wherein DNA sequences from multiple vectors, each comprising at least one expression cassette encoding a part of a heteromultimeric polypeptide and/or at least one selection marker or part thereof flanked by two heterospecific RRSs, are all integrated into a predetermined site of the genome of a mammalian TI host cell. In certain embodiments the selection marker can be partially encoded on the first the vector and partially encoded on the second vector such that only the correct integration of both by double RMCE allows for the expression of the selection marker.

In certain embodiments, targeted integration via recombinase-mediated recombination leads to selection marker and/or the different expression cassettes for the multimeric polypeptide integrated into one or more pre-determined integration sites of a host cell genome free of sequences from a prokaryotic vector.

It has to be pointed out that, as in one embodiment, the SIRT-1 knockout can be performed either before introduction of the exogenous nucleic acid encoding the heterologous polypeptide or thereafter.

### VI. COMPOSITIONS AND METHODS

Herein is reported a method for generating a recombinant mammalian cell expressing a heterologous polypeptide and a method for producing a heterologous polypeptide using said recombinant mammalian cell, wherein in the recombinant mammalian cell the activity/function/expression of the endogenous SIRT-1 gene has been reduced/eliminated/diminished/(completely) knocked-out.

The invention is based, at least in part, on the finding that the knockout of the sirtuin-1 (SIRT-1) gene in mammalian cells, e.g. such as CHO cells, improves recombinant productivity, e.g. of standard IgG-type antibodies and especially of complex antibody formats, and reduces lactate production by the cells during cultivation. Additionally, it has been found that the viability decline at the end of a fed-batch cultivation is reduced, i.e. the timespan with viability above a certain threshold value is increased.

The results obtained by a knockout of the SIRT-1 gene are surprising as the knockout of other genes likewise potentially influencing productivity was without positive effect. Also the combinational knockout of different genes did not perform better than a single SIRT-1 knockout. This was shown in a cell line producing a molecule comprising an antigen binding domain targeting Fibroblast Activation Protein (FAP) and a trimer of 4-1BB ligands (CD137L), named FAP-4-1BBL as reported e.g. in WO 2016/075278 (data presented in the Table below). All cells have the same genotype as the reference cell except for the recited knock-outs.

| **Targeted gene(s)** | **day 14 titer [µg/mL]** |
|---|---|
| SIRT-1 | 4146 |
| SIRT-1+YY1+PTEN | 4137 |
| YY1 | 3606 |
| none (reference) | 3551 (reference) |
| NCK1 | 3431 |
| IFRD1 | 3330 |
| IFRD1+NCK1 | 3302 |
| PTEN | 3165 |
| SIRT-1+HDAC1+HDAC-3 | 1913 |
| HDAC1 | 1826 |
| HDAC3 | 1350 |

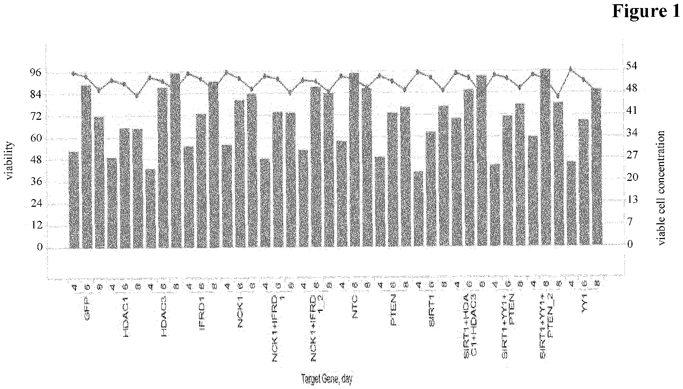

The knockout of the different genes had no effect on cell growth as shown in Figure 1.

Sirtuin 1 (SIRT-1) belongs to the family of sirtuin proteins. SIRT proteins are highly conserved in eukaryotes and are NAD+-dependent enzymes that are involved in the regulation of many cellular pathways (Revollo, J. R. and Li, X. Trends Biochem. Sci. 38 (2013) 160-167.). The SIRT-1 gene encodes an 82 kDa protein that is located in the cytoplasm and nucleus.

Knockout of SIRT-1 gene activity/expression is advantageous in any eukaryotic cell used for the production of heterologous polypeptides, specifically in recombinant CHO cells used or intended to be used to produce recombinant polypeptides, especially antibodies, more specifically in targeted integration recombinant CHO cells. The knockout leads to a significant productivity increase as well as reduction in lactate production as well as an extended cultivation time (reduced/slowed/delayed viability drop). This is of high economic importance for any large scale production process as this results in high yields of product from individual fed-batch processes.

The SIRT-1 knockout is not limited to CHO cells but can also be used in other host cell lines, such as HEK293 cells, CAP cells, and BHK cells.

To knockout SIRT-1 gene activity/expression CRISPR/Cas9 technology has been used. Likewise, any other technology can be employed such as Zinc-Finger-Nucleases or TALENS. In addition, RNA silencing species, such as siRNA/shRNA/miRNA can be employed to knockdown SIRT-1 mRNA levels and as a consequence SIRT-1 gene activity/expression.

Using CRISPR-Cas9 the SIRT-1 gene has been targeted at three different sites using three different gRNAs (see Figure 2) at the same time using multiplexed ribonucleoprotein delivery. Double-strand breaks at the SIRT-1 target sites induce indel formations or due to multiplexed gRNA usage also deletions within the exon 1 sequence (see Figure 3). Sequencing of the PCR-amplified SIRT-1 locus of SIRT-1 knockout cell pools revealed an abrupt interruption of the sequencing reaction at the first gRNA site showing successful targeting for the SIRT-1 gene (see Figure 4). These cell pools consist of a mixture of cells containing unedited, homozygous and heterozygous SIRT-1 loci.

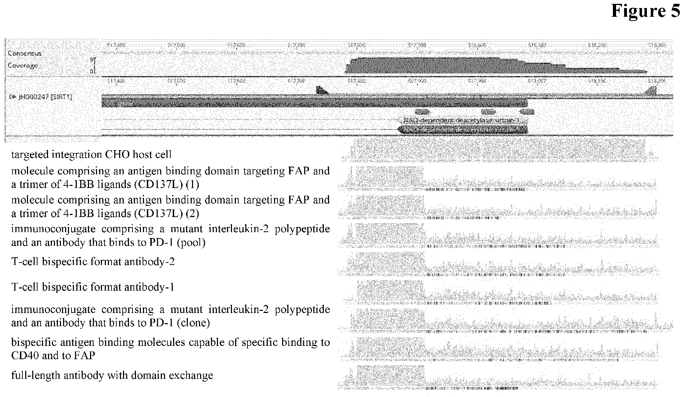

After 28 days of cultivation a re-sequencing has been done showing stability of the knockout (see Figure 5). No growth advantage of wild-type, i.e. cells without SIRT-1 knockout, or growth reduction of the knockout pools, respectively, has been observed.

In a 14-day fed-batch cultivation process, a productivity increase of 2-20% for the SIRT-1 knockout cell pools or clones expressing different complex antibody formats compared to the unmodified cell pools or clones could be observed (data presented in the following Table). The reference cells and the knockout cells have the same genotype except for the additional knockout of the SIRT-1 gene in the knockout cells.

| **antibody** | **titer w/o SIRT-1 knockout (reference)** | **titer w/ SIRT-1 knockout** |
|---|---|---|
| | **[µg/mL]** | **[µg/mL]** |
| molecule comprising an antigen binding domain targeting Fibroblast Activation Protein (FAP) and a trimer of 4-1BB ligands (CD137L) | 3178 | 3527 |
| (see, e.g., WO 2016/075278) | | |
| molecule comprising an antigen binding domain targeting Fibroblast Activation Protein (FAP) and a trimer of 4-1BB ligands (CD137L) | 3259 | 3567 |
| (replica) | | |
| T-cell bispecific format antibody-1 | 3563 | 3566 |
| immunoconjugate comprising a mutant interleukin-2 polypeptide and an antibody that binds to PD-1 (pool) | 2120 | 2495 |
| (see, e.g., WO 2018/184964) | | |
| immunoconjugate comprising a mutant interleukin-2 polypeptide and an antibody that binds to PD-1 (clone) | 2763 | 3411 |
| bispecific antigen binding molecules capable of specific binding to CD40 and to FAP | 2061 | 2146 |
| (see, e.g., WO 2018/185045) | | |
| full-length antibody with domain exchange | 3437 | 3634 |
| T-cell bispecific format antibody-2 | 2467 | 2855 |

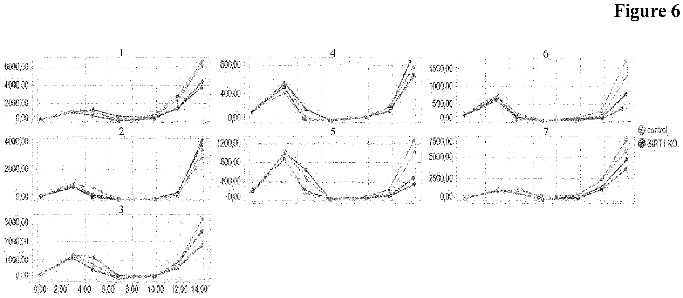

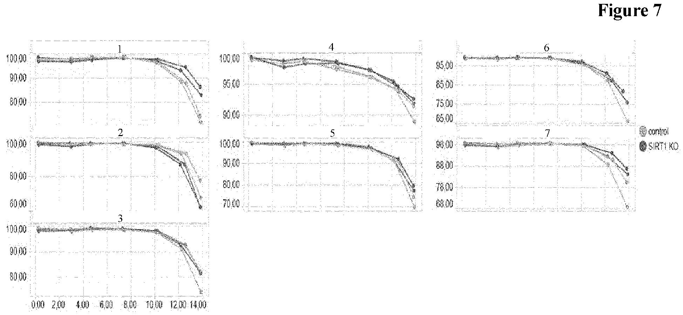

In addition, it has been found that lactate production (see Figure 6) and viability drop (see Figure 7) was similar or slightly reduced in SIRT-1 knockout cells.

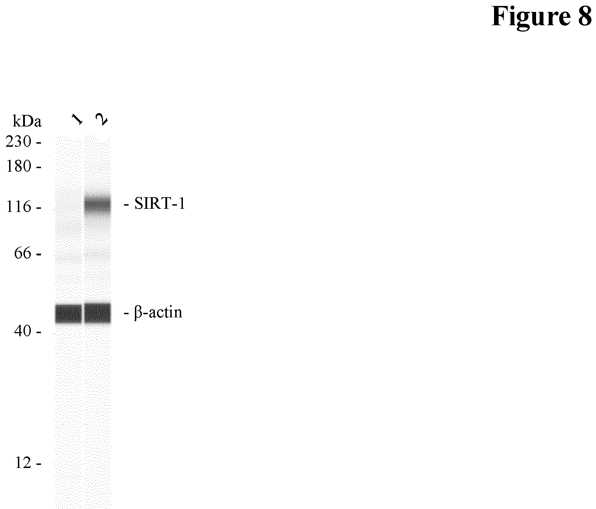

Capillary immunoblotting confirmed abrogated protein levels of sirtuin-1 seven days after ribonucleic particle (RNP) nucleofection (Figure 8).

Without being bound by this theory it is assumed that a homozygous knockout has a more advantageous effect on productivity increase than a heterozygous knockout.

The current invention is summarized below: One independent aspect of the current invention is a mammalian cell wherein the activity/function/expression of the endogenous SIRT-1 gene has been reduced/eliminated/diminished/(completely) knocked-out.

One independent aspect of the current invention is a method for increasing titer/reducing lactate production/extension of cultivation time of a recombinant mammalian cell by reducing/eliminating/diminishing/(completely) knocking-out the activity/function/expression of the endogenous SIRT-1 gene.

One independent aspect of to the current invention is a method for producing a polypeptide comprising the steps of
a) cultivating a mammalian cell comprising a deoxyribonucleic acid encoding the polypeptide optionally under conditions suitable for the expression of the polypeptide, and
b) recovering the polypeptide from the cell or the cultivation medium,
wherein the activity/function/expression of the endogenous SIRT-1 gene has been reduced/eliminated/diminished/(completely) knocked-out.

Another independent aspect of the current invention is a method for producing a recombinant mammalian cell having/with improved/increased recombinant productivity and/or reduced lactate production, wherein the method comprises the following steps:
a) applying a nucleic acid targeting the endogenous SIRT-1 genes in a mammalian cell to reduce/eliminate/diminish/(completely) knock-out the activity/function/expression of the endogenous SIRT-1 gene, and
b) selecting a mammalian cell wherein the activity/function/expression of the endogenous SIRT-1 gene has been reduced/eliminated/diminished/(completely) knocked-out,
thereby producing a recombinant mammalian cell having/with improved/increased recombinant productivity and/or reduced lactate production

In one embodiment of all aspects and embodiments of the current invention the SIRT-1 gene knockout is a heterozygous knockout or a homozygous knockout.

In one embodiment of all aspects and embodiments of the current invention the productivity of the SIRT-1 knockout cell line is at least 10 %, preferably 15 % or more, most preferred 20 % or more increases compared to a SIRT-1 competent parent mammalian cell.

In one embodiment of all aspects and embodiments of the current invention the reduction or elimination or diminishment or knock-out is mediated by a nuclease-assisted gene targeting system. In one embodiment the nuclease-assisted gene targeting system is selected from the group consisting of CRISPR/Cas9, CRISPR/Cpf1, zinc-finger nuclease and TALEN.

In one embodiment of all aspects and embodiments of the current invention the reduction of SIRT-1 gene expression is mediated by RNA silencing. In one embodiment the RNA silencing is selected from the group consisting of siRNA gene targeting and knock-down, shRNA gene targeting and knock-down, and miRNA gene targeting and knock-down.

In one embodiment of all aspects and embodiments of the current invention the SIRT-1 knockout is performed before the introduction of the exogenous nucleic acid encoding the heterologous polypeptide or after the introduction of the exogenous nucleic acid encoding the heterologous polypeptide.

In one embodiment of all aspects and embodiments of the current invention the polypeptide is an antibody. In one embodiment the antibody is an antibody comprising two or more different binding sites and optionally a domain exchange. In one embodiment the antibody comprises three or more binding sites or VH/VL-pairs or Fab fragments and optionally a domain exchange. In one embodiment the antibody is a multispecific antibody.

In one embodiment of all aspects and embodiments of the current invention the polypeptide is an antibody. In one embodiment the antibody is a complex antibody.

In one embodiment of all aspects and embodiments of the current invention the (heterologous) polypeptide is a heterotetrameric polypeptide comprising
- a first heavy chain comprising from N- to C-terminus a first heavy chain variable domain, a CH1 domain, a first light chain variable domain, a CH1 domain, a hinge region, a CH2 domain and a CH3 domain,
- a second heavy chain comprising from N- to C-terminus the first heavy chain variable domain, a CH1 domain, a hinge region, a CH2 domain and a CH3 domain,
- a first light chain comprising from N- to C-terminus a second heavy chain variable domain and a CL domain, and
- a second light chain comprising from N- to C- terminus a second light chain variable domain and a CL domain,
wherein the first heavy chain variable domain and the second light chain variable domain form a first binding site and the second heavy chain variable domain and the first light chain variable domain form a second binding site.

In one embodiment of all aspects and embodiments of the current invention the (heterologous) polypeptide is a heterotetrameric polypeptide comprising
- a first heavy chain comprising from N- to C-terminus a first heavy chain variable domain, a CH1 domain, a second heavy chain variable domain, a CL domain, a hinge region, a CH2 domain and a CH3 domain,
- a second heavy chain comprising from N- to C-terminus the first heavy chain variable domain, a CH1 domain, a hinge region, a CH2 domain and a CH3 domain,
- a first light chain comprising from N- to C-terminus a first light chain variable domain and a CH1 domain, and
- a second light chain comprising from N- to C- terminus a second light chain variable domain and a CL domain,
wherein the first heavy chain variable domain and the second light chain variable domain form a first binding site and the second heavy chain variable domain and the first light chain variable domain form a second binding site.

In one embodiment of all aspects and embodiments of the current invention the (heterologous) polypeptide is a heterotetrameric polypeptide comprising
- a first heavy chain comprising from N- to C-terminus a first heavy chain variable domain, a CH1 domain, a hinge region, a CH2 domain and a CH3 domain,
- a second heavy chain comprising from N- to C-terminus a first light chain variable domain, a CH1 domain, a hinge region, a CH2 domain and a CH3 domain,
- a first light chain comprising from N- to C-terminus a second heavy chain variable domain and a CL domain, and
- a second light chain comprising from N- to C- terminus a second light chain variable domain and a CL domain,
wherein the first heavy chain variable domain and the second light chain variable domain form a first binding site and the second heavy chain variable domain and the first light chain variable domain form a second binding site.

In one embodiment of all aspects and embodiments of the current invention the (heterologous) polypeptide is a heterotetrameric polypeptide comprising
- a first heavy chain comprising from N- to C-terminus a first heavy chain variable domain, a CH1 domain, a hinge region, a CH2 domain and a CH3 domain,
- a second heavy chain comprising from N- to C-terminus a first heavy chain variable domain, a CL domain, a hinge region, a CH2 domain and a CH3 domain,
- a first light chain comprising from N- to C-terminus a first light chain variable domain and a CH1 domain, and
- a second light chain comprising from N- to C- terminus a second light chain variable domain and a CL domain,
wherein the first heavy chain variable domain and the second light chain variable domain form a first binding site and the second heavy chain variable domain and the first light chain variable domain form a second binding site.

In one embodiment of all aspects and embodiments of the current invention the (heterologous) polypeptide is a heteromultimeric polypeptide comprising
- a first heavy chain comprising from N- to C-terminus a first heavy chain variable domain, a CH1 domain, a first heavy chain variable domain, a CH1 domain, a hinge region, a CH2 domain, a CH3 domain and a first light chain variable domain,
- a second heavy chain comprising from N- to C-terminus a first heavy chain variable domain, a CH1 domain, a first heavy chain variable domain, a CH1 domain, a hinge region, a CH2 domain, a CH3 domain and a second heavy chain variable domain, and
- a first light chain comprising from N- to C-terminus a second light chain variable domain and a CL domain,
wherein the first heavy chain variable domain and the second light chain variable domain form a first binding site and the second heavy chain variable domain and the first light chain variable domain form a second binding site.

In one embodiment of all aspects and embodiments of the current invention the (heterologous) polypeptide is a heterotetrameric polypeptide comprising
- a first heavy chain comprising from N- to C-terminus a first heavy chain variable domain, a CH1 domain, a hinge region, a CH2 domain, a CH3 domain, a peptidic linker, a second heavy chain variable domain and a CL domain,
- a second heavy chain comprising from N- to C-terminus a first heavy chain variable domain, a CH1 domain, a hinge region, a CH2 domain and a CH3 domain,
- a first light chain comprising from N- to C-terminus a first light chain variable domain and a CH1 domain, and
- a second light chain comprising from N- to C- terminus a second light chain variable domain and a CL domain,
wherein the second heavy chain variable domain and the first light chain variable domain form a first binding site and the first heavy chain variable domain and the second light chain variable domain form a second binding site.

In one embodiment of all aspects and embodiments of the current invention the (heterologous) polypeptide is a therapeutic antibody. In one preferred embodiment the therapeutic antibody is a bispecific (therapeutic) antibody. In one embodiment the bispecific (therapeutic) antibody is a TCB.

In one embodiment of all aspects and embodiments of the current invention the (heterologous) polypeptide is a bispecific (therapeutic) antibody (TCB) comprising
- a first and a second Fab fragment, wherein each binding site of the first and the second Fab fragment specifically bind to the second antigen,
- a third Fab fragment, wherein the binding site of the third Fab fragment specifically binds to the first antigen, and wherein the third Fab fragment comprises a domain crossover such that the variable light chain domain (VL) and the variable heavy chain domain (VH) are replaced by each other, and
- an Fc-region comprising a first Fc-region polypeptide and a second Fc-region polypeptide,
   wherein the first and the second Fab fragment each comprise a heavy chain fragment and a full length light chain,
   wherein the C-terminus of the heavy chain fragment of the first Fab fragment is fused to the N-terminus of the first Fc-region polypeptide,
   wherein the C-terminus of the heavy chain fragment of the second Fab fragment is fused to the N-terminus of the variable light chain domain of the third Fab fragment and the C-terminus of the heavy chain constant domain 1 of the third Fab fragment is fused to the N-terminus of the second Fc-region polypeptide.

In one embodiment of all aspects and embodiments of the current invention the (heterologous) polypeptide is a trimeric polypeptide comprising
- a first heavy chain comprising from N- to C-terminus a heavy chain variable domain, a CH1 domain, a hinge region, a CH2 domain and a CH3 domain,
- a second heavy chain comprising from N- to C-terminus a heavy chain variable domain, a CH1 domain, a hinge region, a CH2 domain, a CH3 domain, a peptidic linker, and a non-immunoglobulin proteinaceous moiety, and
- a light chain comprising from N- to C-terminus a light chain variable domain and a CL domain,
wherein the heavy chain variable domain and the light chain variable domain form a binding site.

In one embodiment of all aspects and embodiments of the current invention the (heterologous) polypeptide is a trimeric polypeptide comprising
- a first heavy chain comprising from N- to C-terminus a heavy chain variable domain, a CH1 domain, a hinge region, a CH2 domain and a CH3 domain,
- a second heavy chain comprising from N- to C-terminus a non-immunoglobulin proteinaceous moiety, a peptidic linker, a hinge region, a CH2 domain, and a CH3 domain, and
- a light chain comprising from N- to C-terminus a light chain variable domain and a CL domain,
wherein the heavy chain variable domain and the light chain variable domain form a binding site.

Another independent aspect of the current invention is a method for producing a recombinant mammalian cell comprising a deoxyribonucleic acid encoding a polypeptide and secreting the polypeptide comprising the following steps:
a) providing a mammalian cell comprising an exogenous nucleotide sequence integrated at a single site within a locus of the genome of the mammalian cell, wherein the exogenous nucleotide sequence comprises a first and a second recombination recognition sequence flanking at least one first selection marker, and a third recombination recognition sequence located between the first and the second recombination recognition sequence, and all the recombination recognition sequences are different;
b) introducing into the cell provided in a) a composition of two deoxyribonucleic acids comprising three different recombination recognition sequences and one to eight expression cassettes, wherein
   the first deoxyribonucleic acid comprises in 5'- to 3'-direction,
      - a first recombination recognition sequence,
      - one or more expression cassette(s),
      - a 5'-terminal part of an expression cassette encoding one second selection marker, and
      - a first copy of a third recombination recognition sequence,
      and
   the second deoxyribonucleic acid comprises in 5'- to 3'-direction
      - a second copy of the third recombination recognition sequence,
      - a 3'-terminal part of an expression cassette encoding the one second selection marker,
      - one or more expression cassette(s), and
      - a second recombination recognition sequence,
   wherein the first to third recombination recognition sequences of the first and second deoxyribonucleic acids are matching the first to third recombination recognition sequence on the integrated exogenous nucleotide sequence,
   wherein the 5'-terminal part and the 3'-terminal part of the expression cassette encoding the one second selection marker when taken together form a functional expression cassette of the one second selection marker;
c) introducing
   i) either simultaneously with the first and second deoxyribonucleic acid of b); or
   ii) sequentially thereafter
      one or more recombinase,
      wherein the one or more recombinases recognize the recombination recognition sequences of the first and the second deoxyribonucleic acid; (and optionally wherein the one or more recombinases perform two recombinase mediated cassette exchanges;)
         and
d) selecting for cells expressing the second selection marker and secreting the polypeptide,
   thereby producing a recombinant mammalian cell comprising a deoxyribonucleic acid encoding the polypeptide and secreting the polypeptide.

In one embodiment of all aspects and embodiments of the current invention the recombinase is Cre recombinase.

In one embodiment of all aspects and embodiments of the current invention the deoxyribonucleic acid is stably integrated into the genome of the mammalian cell at a single site or locus.

In one embodiment of all aspects and embodiments of the current invention the deoxyribonucleic acid encoding the polypeptide comprises one to eight expression cassettes.

In one embodiment of all aspects and embodiments of the current invention the deoxyribonucleic acid encoding the polypeptide comprises at least 4 expression cassettes wherein
- a first recombination recognition sequence is located 5' to the most 5' (i.e. first) expression cassette,
- a second recombination recognition sequence is located 3' to the most 3' expression cassette, and
- a third recombination recognition sequence is located
- between the first and the second recombination recognition sequence, and
- between two of the expression cassettes,
and
wherein all recombination recognition sequences are different.

In one embodiment of all aspects and embodiments of the current invention the third recombination recognition sequence is located between the fourth and the fifth expression cassette.

In one embodiment of all aspects and embodiments of the current invention the deoxyribonucleic acid encoding the polypeptide comprises a further expression cassette encoding for a selection marker.

In one embodiment of all aspects and embodiments of the current invention the deoxyribonucleic acid encoding the polypeptide comprises a further expression cassette encoding for a selection marker and the expression cassette encoding for the selection marker is located partly 5' and partly 3' to the third recombination recognition sequence, wherein the 5'-located part of said expression cassette comprises the promoter and the start-codon and the 3'-located part of said expression cassette comprises the coding sequence without a start-codon and a polyA signal, wherein the start-codon is operably linked to the coding sequence.

In one embodiment of all aspects and embodiments of the current invention the expression cassette encoding for a selection marker is located either
i) 5', or
ii) 3', or
iii) partly 5' and partly 3'
to the third recombination recognition sequence.

In one embodiment of all aspects and embodiments of the current invention the expression cassette encoding for a selection marker is located partly 5' and partly 3' to the third recombination recognition sequences, wherein the 5'-located part of said expression cassette comprises the promoter and a start-codon and the 3'-located part of said expression cassette comprises the coding sequence without a start-codon and a polyA signal.

In one embodiment of all aspects and embodiments of the current invention the 5'-located part of the expression cassette encoding the selection marker comprises a promoter sequence operably linked to a start-codon, whereby the promoter sequence is flanked upstream by (i.e. is positioned downstream to) the second, third or fourth, respectively, expression cassette and the start-codon is flanked downstream by (i.e. is positioned upstream of) the third recombination recognition sequence; and the 3'-located part of the expression cassette encoding the selection marker comprises a nucleic acid encoding the selection marker lacking a start-codon and is flanked upstream by the third recombination recognition sequence and downstream by the third, fourth or fifth, respectively, expression cassette.

In one embodiment of all aspects and embodiments of the current invention the start-codon is a translation start-codon. In one embodiment the start-codon is ATG.

In one embodiment of all aspects and embodiments of the current invention the first deoxyribonucleic acid is integrated into a first vector and the second deoxyribonucleic acid is integrated into a second vector.

In one embodiment of all aspects and embodiments of the current invention each of the expression cassettes comprise in 5'-to-3' direction a promoter, a coding sequence and a polyadenylation signal sequence optionally followed by a terminator sequence.

In one embodiment of all aspects and embodiments of the current invention the promoter is the human CMV promoter with or without intron A, the polyadenylation signal sequence is the bGH polyA site and the terminator is the hGT terminator.

In one embodiment of all aspects and embodiments of the current invention the promoter is the human CMV promoter with intron A, the polyadenylation signal sequence is the bGH polyadenylation signal sequence and the terminator is the hGT terminator except for the expression cassette of the selection marker, wherein the promoter is the SV40 promoter and the polyadenylation signal sequence is the SV40 polyadenylation signal sequence and a terminator is absent.

In one embodiment of all aspects and embodiments of the current invention the mammalian cell is a CHO cell. In one embodiment the CHO cell is a CHO-K1 cell.

In one embodiment of all aspects and embodiments of the current invention the polypeptide is selected from the group of polypeptides consisting of a bivalent, monospecific antibody, a bivalent, bispecific antibody comprising at least one domain exchange, and a trivalent, bispecific antibody comprising at least one domain exchange.

In one embodiment of all previous aspects and embodiments of the current invention the recombinase recognition sequences are L3, 2L and LoxFas. In one embodiment L3 has the sequence of SEQ ID NO: 01, 2L has the sequence of SEQ ID NO: 02 and LoxFas has the sequence of SEQ ID NO: 03. In one embodiment the first recombinase recognition sequence is L3, the second recombinase recognition sequence is 2L and the third recombinase recognition sequence is LoxFas.

In one embodiment of all previous aspects and embodiments of the current invention the promoter is the human CMV promoter with intron A, the polyadenylation signal sequence is the bGH polyA site and the terminator sequence is the hGT terminator.

In one embodiment of all previous aspects and embodiments of the current invention the promoter is the human CMV promoter with intron A, the polyadenylation signal sequence is the bGH polyA site and the terminator sequence is the hGT terminator except for the expression cassette(s) of the selection marker(s), wherein the promoter is the SV40 promoter and the polyadenylation signal sequence is the SV40 polyA site and a terminator sequence is absent.

In one embodiment of all previous aspects and embodiments of the current invention the human CMV promoter has the sequence of SEQ ID NO: 04. In one embodiment the human CMV promoter has the sequence of SEQ ID NO: 06.

In one embodiment of all previous aspects and embodiments of the current invention the bGH polyadenylation signal sequence is SEQ ID NO: 08.

In one embodiment of all previous aspects and embodiments of the current invention the hGT terminator has the sequence of SEQ ID NO: 09.

In one embodiment of all previous aspects and embodiments of the current invention the SV40 promoter has the sequence of SEQ ID NO: 10.

In one embodiment of all previous aspects and embodiments of the current invention the SV40 polyadenylation signal sequence is SEQ ID NO: 07.

The following examples, sequences and figures are provided to aid the understanding of the present invention, the true scope of which is set forth in the appended claims. It is understood that modifications can be made in the procedures set forth without departing from the spirit of the invention.

### Description of the Sequences

| | |
|---|---|
| SEQ ID NO: 01: | exemplary sequence of an L3 recombinase recognition sequence |
| SEQ ID NO: 02: | exemplary sequence of a 2L recombinase recognition sequence |
| SEQ ID NO: 03: | exemplary sequence of a LoxFas recombinase recognition sequence |
| SEQ ID NO: 04-06: | exemplary variants of human CMV promoter |
| SEQ ID NO: 07: | exemplary SV40 polyadenylation signal sequence |
| SEQ ID NO: 08: | exemplary bGH polyadenylation signal sequence |
| SEQ ID NO: 09: | exemplary hGT terminator sequence |
| SEQ ID NO: 10: | exemplary SV40 promoter sequence |
| SEQ ID NO: 11: | exemplary GFP nucleic acid sequence |
| SEQ ID NO: 12: | gRNA_SIRT1_1: TATCATCCAACTCAGGTGGA |
| SEQ ID NO: 13: | gRNA_SIRT1_2: GCAGCATCTCATGATTGGCA |
| SEQ ID NO: 14: | gRNA_SIRT1_3: GCATTCTTGAAGTAACTTCA |
| SEQ ID NO: 15: | oSA060_SIRT1_for: GCTGCCCTTCAAGTTATGGC |
| SEQ ID NO: 16: | oSA061_SIRT1_rev: GCTGGCCTTTTGACTCACAG |
| SEQ ID NO: 17: | amino acid sequence of human sirtuin-1 |
| SEQ ID NO: 18: | amino acid sequence of chinese hamster sirtuin-1 |

### Descrintion of the Figures

- **Figure 1**: Growth & Viability after CRISPR/Cas9-based knockout (KO) of target genes. Shown is viable cell count of engineered clones of a cell expressing an antibody comprising an antigen binding domain targeting Fibroblast Activation Protein (FAP) and a trimer of 4-1BB ligands (CD137L) with different CRISPR RNP-based target gene knockouts. NTC: non-targeting control gRNA; line = viability; bars = viable cell concentration.

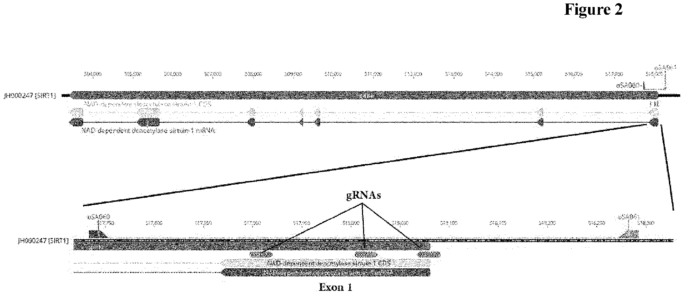

- **Figure 2**: SIRT-1 gene derived from public CHO genome. (A) SIRT-1 gene showing exons and introns, gRNA target sites (blue) and primer sequences (oSA060 and oSA061) for SIRT-1 amplicon preparation (green). (B) Zoom on exon 1 of SIRT-1 gene.

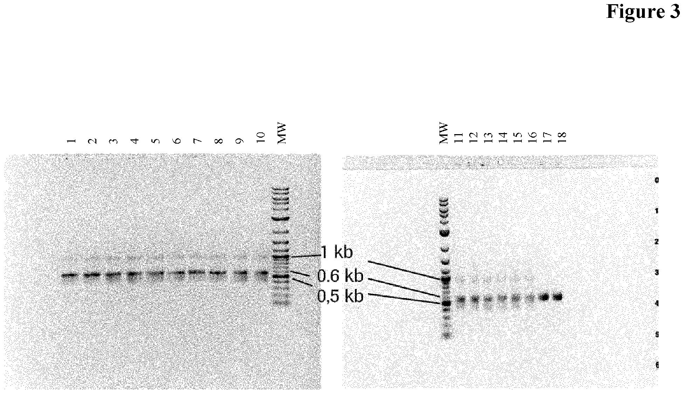

- **Figure 3**: Agarose gel electrophoresis of PCR amplification of SIRT-1 gene locus. Indels have been detected.
Lane 1= bispecific antigen binding molecules capable of specific binding to CD40 and to FAP; lane 2 = replica of lane 1; lane 3 = molecule comprising an antigen binding domain targeting Fibroblast Activation Protein (FAP) and a trimer of 4-1BB ligands (CD137L) (1); lane 4 = replica of lane 3; lane 5 = molecule comprising an antigen binding domain targeting Fibroblast Activation Protein (FAP) and a trimer of 4-1BB ligands (CD137L) (2); lane 6 = replica of lane 5; lane 7 = T-cell bispecific format antibody-1; lane 8 = replica of lane 7; lane 9 = full-length antibody with domain exchange; lane 10 = replica of lane 9; lane 11 = immunoconjugate comprising a mutant interleukin-2 polypeptide and an antibody that binds to PD-1 (pool); lane 12 = replica of lane 11; lane 13 = immunoconjugate comprising a mutant interleukin-2 polypeptide and an antibody that binds to PD-1 (clone); lane 14 = replica of lane 13; lane 15 = T-cell bispecific format antibody-2; lane 16 = replica of lane 15; lane 17 = targeted integration CHO host cell; lane 18 = replica of lane 17; MW = molecular weight marker.

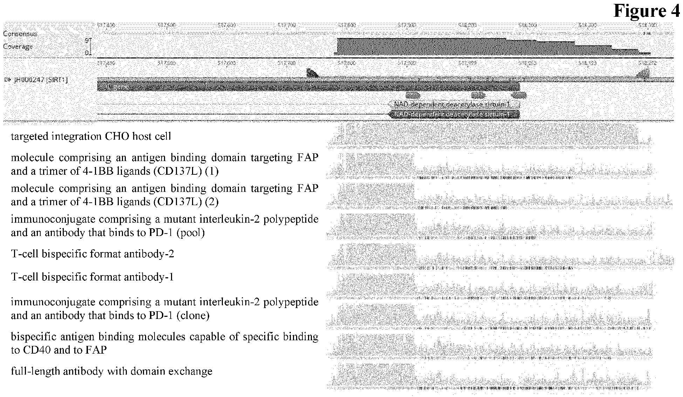

- **Figure 4**: Sequencing verification of SIRT-1 knockout in multiplexed CRISPR/Cas9-modified cell pools. Comparison of sequencing results of a SIRT-1 gene amplicon of unmodified pools/clones to SIRT-1 knockout pools/clones (containing mixture of unmodified/heterozygous/homozygous SIRT-1 knockout loci) for six different complex antibody formats.
- **Figure 5**: Sanger re-sequencing of SIRT-1 locus. SIRT-1 stably remains disrupted on pool level in all cell lines 28 days post knockout (Cas9-gRNA RNP transfection).
- **Figure 6**: Fed-batch lactate data [mg/l]. Comparison of unmodified pools / clones to SIRT-1 knockout pools/ clones (containing mixture of unmodified/heterozygous/homozygous SIRT-1 knockout loci) for six different complex antibody formats.
Cell expressing
1 = T-cell bispecific format antibody-2; 2 = T-cell bispecific format antibody-1; 3 = full-length antibody with domain exchange; 4 = molecule comprising an antigen binding domain targeting FAP and a trimer of 4-1BB ligands (CD137L); 5 = immunoconjugate comprising a mutant interleukin-2 polypeptide and an antibody that binds to PD-1 (pool), 6 = bispecific antigen binding molecules capable of specific binding to CD40 and to FAP; 7 = immunoconjugate comprising a mutant interleukin-2 polypeptide and an antibody that binds to PD-1 (clone).
- **Figure 7**: Fed-batch viability data [%]. Comparison of unmodified pools / clones to SIRT-1 knockout pools/ clones (containing mixture of unmodified/heterozygous/homozygous SIRT-1 knockout loci) for six different complex antibody formats.
Cell expressing
1 = T-cell bispecific format antibody-2; 2 = T-cell bispecific format antibody-1; 3 = full-length antibody with domain exchange; 4 = molecule comprising an antigen binding domain targeting FAP and a trimer of 4-1BB ligands (CD137L); 5 = immunoconjugate comprising a mutant interleukin-2 polypeptide and an antibody that binds to PD-1 (pool), 6 = bispecific antigen binding molecules capable of specific binding to CD40 and to FAP; 7 = immunoconjugate comprising a mutant interleukin-2 polypeptide and an antibody that binds to PD-1 (clone).
- **Figure 8**: Capillary Immunoblotting confirms abrogated protein levels 7 days after RNP nucleofection. 1= Host cell line was nucleofected with 5 pmol of Cas9 protein and 5 pmol of 3 gRNAs targeting the SIRT-1 genomic locus; 2 = Host cell line was nucleofected with 5 pmol of Cas9 protein.

### Examples

### Example 1

### General techniques

### 1) Recombinant DNA techniques

Standard methods were used to manipulate DNA as described in Sambrook et al., Molecular Cloning: A Laboratory Manual, Second Edition, Cold Spring Harbor Laboratory Press, Cold Spring Harbor, N.Y, (1989). The molecular biological reagents were used according to the manufacturer's instructions.

### 2) DNA sequence determination

DNA sequencing was performed at SequiServe GmbH (Vaterstetten, Germany)

### 3) DNA and protein sequence analysis and sequence data management

The EMBOSS (European Molecular Biology Open Software Suite) software package and Invitrogen's Vector NTI version 11.5 were used for sequence creation, mapping, analysis, annotation and illustration.

### 4) Gene and oligonucleotide synthesis

Desired gene segments were prepared by chemical synthesis at Geneart GmbH (Regensburg, Germany). The synthesized gene fragments were cloned into an E. coli plasmid for propagation/amplification. The DNA sequences of subcloned gene fragments were verified by DNA sequencing. Alternatively, short synthetic DNA fragments were assembled by annealing chemically synthesized oligonucleotides or via PCR. The respective oligonucleotides were prepared by metabion GmbH (Planegg-Martinsried, Germany).

### 5) Reagents

All commercial chemicals, antibodies and kits were used as provided according to the manufacturer's protocol if not stated otherwise.

### 6) Cultivation of TI host cell line

TI CHO host cells were cultivated at 37°C in a humidified incubator with 85% humidity and 5% CO₂. They were cultivated in a proprietary DMEM/F12-based medium containing 300 µg/ml Hygromycin B and 4 µg/ml of a second selection marker. The cells were splitted every 3 or 4 days at a concentration of 0.3x10E6 cells/ml in a total volume of 30 ml. For the cultivation 125 ml non-baffle Erlenmeyer shake flasks were used. Cells were shaken at 150 rpm with a shaking amplitude of 5 cm. The cell count was determined with Cedex HiRes Cell Counter (Roche). Cells were kept in culture until they reached an age of 60 days.

### 7) Cloning

### General

Cloning with R-sites depends on DNA sequences next to the gene of interest (GOI) that are equal to sequences lying in following fragments. Like that, assembly of fragments is possible by overlap of the equal sequences and subsequent sealing of nicks in the assembled DNA by a DNA ligase. Therefore, a cloning of the single genes in particular preliminary vectors containing the right R-sites is necessary. After successful cloning of these preliminary vectors the gene of interest flanked by the R-sites is cut out via restriction digest by enzymes cutting directly next to the R-sites. The last step is the assembly of all DNA fragments in one step. In more detail, a 5'-exonuclease removes the 5'-end of the overlapping regions (R-sites). After that, annealing of the R-sites can take place and a DNA polymerase extends the 3'-end to fill the gaps in the sequence. Finally, the DNA ligase seals the nicks in between the nucleotides. Addition of an assembly master mix containing different enzymes like exonucleases, DNA polymerases and ligases, and subsequent incubation of the reaction mix at 50°C leads to an assembly of the single fragments to one plasmid. After that, competent E. coli cells are transformed with the plasmid.

For some vectors, a cloning strategy via restriction enzymes was used. By selection of suitable restriction enzymes, the wanted gene of interest can be cut out and afterwards inserted into a different vector by ligation. Therefore, enzymes cutting in a multiple cloning site (MCS) are preferably used and chosen in a smart manner, so that a ligation of the fragments in the correct array can be conducted. If vector and fragment are previously cut with the same restriction enzyme, the sticky ends of fragment and vector fit perfectly together and can be ligated by a DNA ligase, subsequently. After ligation, competent E. coli cells are transformed with the newly generated plasmid.

### Cloning via Restriction digestion

For the digest of plasmids with restriction enzymes the following components were pipetted together on ice:

**Table: Restriction Digestion Reaction Mix**

| **component** | **ng (set point)** | **µl** |
|---|---|---|
| purified DNA | tbd | tbd |
| CutSmart Buffer (10x) | | 5 |
| Restriction Enzyme | | 1 |
| PCR-grade Water | | ad 50 |
| Total | | 50 |

If more enzymes were used in one digestion, 1 µl of each enzyme was used and the volume adjusted by addition of more or less PCR-grade water. All enzymes were selected on the preconditions that they are qualified for the use with CutSmart buffer from new England Biolabs (100% activity) and have the same incubation temperature (all 37°C).

Incubation was performed using thermomixers or thermal cyclers, allowing to incubate the samples at a constant temperature (37°C). During incubation the samples were not agitated. Incubation time was set at 60 min. Afterwards the samples were directly mixed with loading dye and loaded onto an agarose electrophoresis gel or stored at 4°C/on ice for further use.

A 1% agarose gel was prepared for gel electrophoresis. Therefor 1.5 g of multipurpose agarose were weighed into a 125 Erlenmeyer shake flask and filled up with 150 ml TAE-buffer. The mixture was heated up in a microwave oven until the agarose was completely dissolved. 0.5 µg/ml ethidium bromide were added into the agarose solution. Thereafter the gel was cast in a mold. After the agarose was set, the mold was placed into the electrophoresis chamber and the chamber filled with TAE-buffer. Afterwards the samples were loaded. In the first pocket (from the left) an appropriate DNA molecular weight marker was loaded, followed by the samples. The gel was run for around 60 minutes at <130V. After electrophoresis the gel was removed from the chamber and analyzed in an UV-Imager.

The target bands were cut and transferred to 1.5 ml Eppendorf tubes. For purification of the gel, the QIAquick Gel Extraction Kit from Qiagen was used according to the manufacturer's instructions. The DNA fragments were stored at -20°C for further use.

The fragments for the ligation were pipetted together in a molar ratio of 1:2, 1:3 or 1:5 vector to insert, depending on the length of the inserts and the vector-fragments and their correlation to each other. If the fragment, that should be inserted into the vector was short, a 1:5-ratio was used. If the insert was longer, a smaller amount of it was used in correlation to the vector. An amount of 50 ng of vector were used in each ligation and the particular amount of insert calculated with NEBioCalculator. For ligation, the T4 DNA ligation kit from NEB was used. An example for the ligation mixture is depicted in the following Table:

**Table: Ligation Reaction Mix**

| **component** | **ng (set point)** | **conc. [ng/ul]** | **µl** |
|---|---|---|---|
| T4 DNA Ligase Buffer (10x) | | | 2 |
| Vector DNA (4000 bp) | 50 | 50 | 1 |
| Insert DNA (2000 bp) | 125 | 20 | 6.25 |
| Nuclease-free Water | | | 9.75 |
| T4 Ligase | | | 1 |
| Total | | | 20 |

All components were pipetted together on ice, starting with the mixing of DNA and water, addition of buffer and finally addition of the enzyme. The reaction was gently mixed by pipetting up and down, briefly microfuged and then incubated at room temperature for 10 minutes. After incubation, the T4 ligase was heat inactivated at 65°C for 10 minutes. The sample was chilled on ice. In a final step, 10-beta competent E. coli cells were transformed with 2 µl of the ligated plasmid (see below).

### Cloning via R-site assembly

For assembly, all DNA fragments with the R-sites at each end were pipetted together on ice. An equimolar ratio (0.05 ng) of all fragments was used, as recommended by the manufacturer, when more than 4 fragments are being assembled. One half of the reaction mix was embodied by NEBuilder HiFi DNA Assembly Master Mix. The total reaction volume was 40 µl and was reached by a fill-up with PCR-clean water. In the following Table an exemplary pipetting scheme is depicted.

**Table: Assembly Reaction Mix**

| **component** | **bp** | **pmol (set point)** | **ng (set point)** | **conc. [ng/µl]** | **µl** |
|---|---|---|---|---|---|
| Insert 1 | 2800 | 0.05 | 88.9 | 21 | 4.23 |
| Insert 2 | 2900 | 0.05 | 90.5 | 35 | 2.59 |
| Insert 3 | 4200 | 0.05 | 131.6 | 35.5 | 3.71 |
| Insert 4 | 3600 | 0.05 | 110.7 | 23 | 4.81 |
| Vector | 4100 | 0.05 | 127.5 | 57.7 | 2.21 |
| NEBuilder HiFi DNA Assembly Master Mix | | | | | 20 |
| PCR-clean Water | | | | | 2.45 |
| Total | | | | | 40 |

After set up of the reaction mixture, the tube was incubated in a thermocycler at constantly 50°C for 60 minutes. After successful assembly, 10-beta competent E. coli bacteria were transformed with 2 µl of the assembled plasmid DNA (see below).

### Transformation 10-beta competent E. coli cells

For transformation the 10-beta competent E. coli cells were thawed on ice. After that, 2 µl of plasmid DNA were pipetted directly into the cell suspension. The tube was flicked and put on ice for 30 minutes. Thereafter, the cells were placed into the 42°C-warm thermal block and heat-shocked for exactly 30 seconds. Directly afterwards, the cells were chilled on ice for 2 minutes. 950 µl of NEB 10-beta outgrowth medium were added to the cell suspension. The cells were incubated under shaking at 37°C for one hour. Then, 50-100 µl were pipetted onto a pre-warmed (37°C) LB-Amp agar plate and spread with a disposable spatula. The plate was incubated overnight at 37°C. Only bacteria which have successfully incorporated the plasmid, carrying the resistance gene against ampicillin, can grow on this plates. Single colonies were picked the next day and cultured in LB-Amp medium for subsequent plasmid preparation.

### Bacterial culture

Cultivation of E. coli was done in LB-medium, short for Luria Bertani, that was spiked with 1 ml/L 100 mg/ml ampicillin resulting in an ampicillin concentration of 0.1 mg/ml. For the different plasmid preparation quantities, the following amounts were inoculated with a single bacterial colony.

**Table: E. coli cultivation volumes**

| **Quantity plasmid preparation** | **Volume LB-Amp medium [ml]** | **Incubation time [h]** |
|---|---|---|
| Mini-Prep 96-well (EpMotion) | 1.5 | 23 |
| Mini-Prep 15 ml-tube | 3.6 | 23 |
| Maxi-Prep | 200 | 16 |

For Mini-Prep, a 96-well 2 ml deep-well plate was filled with 1.5 ml LB-Amp medium per well. The colonies were picked and the toothpick was tuck in the medium. When all colonies were picked, the plate closed with a sticky air porous membrane. The plate was incubated in a 37°C incubator at a shaking rate of 200 rpm for 23 hours.

For Mini-Preps a 15 ml-tube (with a ventilated lid) was filled with 3.6 ml LB-Amp medium and equally inoculated with a bacterial colony. The toothpick was not removed but left in the tube during incubation. Like the 96-well plate the tubes were incubated at 37°C, 200 rpm for 23 hours.

For Maxi-Prep 200 ml of LB-Amp medium were filled into an autoclaved glass 1 L Erlenmeyer flask and inoculated with 1 ml of bacterial day-culture, that was roundabout 5 hours old. The Erlenmeyer flask was closed with a paper plug and incubated at 37°C, 200 rpm for 16 hours.

### Plasmid preparation

For Mini-Prep, 50 µl of bacterial suspension were transferred into a 1 ml deep-well plate. After that, the bacterial cells were centrifuged down in the plate at 3000 rpm, 4°C for 5 min. The supernatant was removed and the plate with the bacteria pellets placed into an EpMotion. After ca. 90 minutes the run was done and the eluted plasmid-DNA could be removed from the EpMotion for further use.

For Mini-Prep, the 15 ml tubes were taken out of the incubator and the 3.6 ml bacterial culture splitted into two 2 ml Eppendorf tubes. The tubes were centrifuged at 6,800xg in a table-top microcentrifuge for 3 minutes at room temperature. After that, Mini-Prep was performed with the Qiagen QIAprep Spin Miniprep Kit according to the manufacturer's instructions. The plasmid DNA concentration was measured with Nanodrop.

Maxi-Prep was performed using the Macherey-Nagel NucleoBond^{®} Xtra Maxi EF Kit according to the manufacturer's instructions. The DNA concentration was measured with Nanodrop.

### Ethanol precipitation

The volume of the DNA solution was mixed with the 2.5-fold volume ethanol 100%. The mixture was incubated at -20°C for 10 min. Then the DNA was centrifuged for 30 min. at 14,000 rpm, 4°C. The supernatant was carefully removed and the pellet washed with 70% ethanol. Again, the tube was centrifuged for 5 min. at 14,000 rpm, 4°C. The supernatant was carefully removed by pipetting and the pellet dried. When the ethanol was evaporated, an appropriate amount of endotoxin-free water was added. The DNA was given time to re-dissolve in the water overnight at 4°C. A small aliquot was taken and the DNA concentration was measured with a Nanodrop device.

### Example 2

### Plasmid generation

### Expression cassette composition

For the expression of an antibody chain a transcription unit comprising the following functional elements was used:
- the immediate early enhancer and promoter from the human cytomegalovirus including intron A,
- a human heavy chain immunoglobulin 5'-untranslated region (5'UTR),
- a murine immunoglobulin heavy chain signal sequence,
- a nucleic acid encoding the respective antibody chain,
- the bovine growth hormone polyadenylation sequence (BGH pA), and
- optionally the human gastrin terminator (hGT).

Beside the expression unit/cassette including the desired gene to be expressed the basic/standard mammalian expression plasmid contains
- an origin of replication from the vector pUC18 which allows replication of this plasmid in E. coli, and
- a beta-lactamase gene which confers ampicillin resistance in E. coli.

### Front- and back-vector cloning

To construct two-plasmid antibody constructs, antibody HC and LC fragments were cloned into a front vector backbone containing L3 and LoxFAS sequences, and a back vector containing LoxFAS and 2L sequences and a pac selectable marker. The Cre recombinase plasmid pOG231 (Wong, E.T., et al., Nuc. Acids Res. 33 (2005) e147; O'Gorman, S., et al., Proc. Natl. Acad. Sci. USA 94 (1997) 14602-14607) was used for all RMCE processes.

The cDNAs encoding the respective antibody chains were generated by gene synthesis (Geneart, Life Technologies Inc.). The gene synthesis and the backbone-vectors were digested with HindIII-HF and EcoRI-HF (NEB) at 37 °C for 1 h and separated by agarose gel electrophoresis. The DNA-fragment of the insert and backbone were cut out from the agarose gel and extracted by QIAquick Gel Extraction Kit (Qiagen). The purified insert and backbone fragment was ligated via the Rapid Ligation Kit (Roche) following the manufacturer's protocol with an Insert/Backbone ratio of 3:1. The ligation approach was then transformed in competent E.coli DH5α via heat shock for 30 sec. at 42 °C and incubated for 1 h at 37 °C before they were plated out on agar plates with ampicillin for selection. Plates were incubated at 37 °C overnight.

On the following day clones were picked and incubated overnight at 37 °C under shaking for the Mini or Maxi-Preparation, which was performed with the EpMotion^{®} 5075 (Eppendorf) or with the QIAprep Spin Mini-Prep Kit (Qiagen)/ NucleoBond Xtra Maxi EF Kit (Macherey & Nagel), respectively. All constructs were sequenced to ensure the absence of any undesirable mutations (SequiServe GmbH).

In the second cloning step, the previously cloned vectors were digested with KpnI-HF/SalI-HF and SalI-HF/MfeI-HF with the same conditions as for the first cloning. The TI backbone vector was digested with KpnI-HF and MfeI - HF. Separation and extraction was performed as described above. Ligation of the purified insert and backbone was performed using T4 DNA Ligase (NEB) following the manufacturing protocol with an Insert/Insert/Backbone ratio of 1:1:1 overnight at 4 °C and inactivated at 65 °C for 10 min. The following cloning steps were performed as described above.

The cloned plasmids were used for the TI transfection and pool generation.

### Example 3

### Cultivation, transfection, selection and single cell cloning

TI host cells were propagated in disposable 125 ml vented shake flasks under standard humidified conditions (95% rH, 37°C, and 5% CO₂) at a constant agitation rate of 150 rpm in a proprietary DMEM/F12-based medium. Every 3-4 days the cells were seeded in chemically defined medium containing selection marker 1 and selection marker 2 in effective concentrations with a concentration of 3x10E5 cells/ml. Density and viability of the cultures were measured with a Cedex HiRes cell counter (F. Hoffmann-La Roche Ltd, Basel, Switzerland).

For stable transfection, equimolar amounts of front and back vector were mixed. 1 µg Cre expression plasmid was added per 5 µg of the mixture, i.e. 5 µg Cre expression plasmid or Cre mRNA was added to 25 µg of the front- and back-vector mixture.

Two days prior to transfection TI host cells were seeded in fresh medium with a density of 4x10E5 cells/ml. Transfection was performed with the Nucleofector device using the Nucleofector Kit V (Lonza, Switzerland), according to the manufacturer's protocol. 3x10E7 cells were transfected with a total of 30 µg nucleic acids, i.e. either with 30 µg plasmid (5 µg Cre plasmid and 25 µg front- and back-vector mixture) or with 5 µg Cre mRNA and 25 µg front-and back-vector mixture. After transfection the cells were seeded in 30 ml medium without selection agents.

On day 5 after seeding the cells were centrifuged and transferred to 80 mL chemically defined medium containing puromycin (selection agent 1) and 1-(2'-deoxy-2'-fluoro-1-beta-D-arabinofuranosyl-5-iodo)uracil (FIAU; selection agent 2) at effective concentrations at 6x10E5 cells/ml for selection of recombinant cells. The cells were incubated at 37 °C, 150 rpm. 5% CO2, and 85% humidity from this day on without splitting. Cell density and viability of the culture was monitored regularly. When the viability of the culture started to increase again, the concentrations of selection agents 1 and 2 were reduced to about half the amount used before. In more detail, to promote the recovering of the cells, the selection pressure was reduced if the viability is > 40 % and the viable cell density (VCD) is > 0.5x10E6 cells/mL. Therefore, 4x10E5 cells/ml were centrifuged and resuspended in 40 ml selection media II (chemically-defined medium, ½ selection marker 1 & 2). The cells were incubated with the same conditions as before and also not splitted.

Ten days after starting selection, the success of Cre mediated cassette exchange was checked by flow cytometry measuring the expression of intracellular GFP and extracellular heterologous polypeptide bound to the cell surface. An APC antibody (allophycocyanin-labeled F(ab')2 Fragment goat anti-human IgG) against human antibody light and heavy chain was used for FACS staining. Flow cytometry was performed with a BD FACS Canto II flow cytometer (BD, Heidelberg, Germany). Ten thousand events per sample were measured. Living cells were gated in a plot of forward scatter (FSC) against side scatter (SSC). The live cell gate was defined with non-transfected TI host cells and applied to all samples by employing the FlowJo 7.6.5 EN software (TreeStar, Olten, Switzerland). Fluorescence of GFP was quantified in the FITC channel (excitation at 488 nm, detection at 530 nm). Heterologous polypeptide was measured in the APC channel (excitation at 645 nm, detection at 660 nm). Parental CHO cells, i.e. those cells used for the generation of the TI host cell, were used as a negative control with regard to GFP and [[X]] expression. Fourteen days after the selection had been started, the viability exceeded 90% and selection was considered as complete.

After selection, the pool of stably transfected cells was subjected to single-cell cloning by limiting dilution. For this purpose, cells were stained with Cell Tracker Green^{™} (Thermo Fisher Scientific, Waltham, MA) and plated in 384-well plates with 0.6 cells/well. For single-cell cloning and all further cultivation steps selection agent 2 was omitted from the medium. Wells containing only one cell were identified by bright field and fluorescence based plate imaging. Only wells that contained one cell were further considered. Approximately three weeks after plating colonies were picked from confluent wells and further cultivated in 96-well plates.

After four days in 96-well plates, the antibody titers in the culture medium were measured with an anti-human IgG sandwich ELISA. In brief, antibodies were captured from the cell culture fluid with an anti-human Fc antibody bound to a MaxiSorp microtiter plate (Nunc^{™}, Sigma-Aldrich) and detected with an anti-human Fc antibody-POD conjugate which binds to an epitope different from the capture antibody. The secondary antibody was quantified by chemiluminescence employing the BM Chemiluminescence ELISA Substrate (POD) (Sigma-Aldrich).

### Example 4

### FACS screening

FACS analysis was performed to check the transfection efficiency and the RMCE efficiency of the transfection. 4x10E5 cells of the transfected approaches were centrifuged (1200 rpm, 4 min.) and washed twice with 1 mL PBS. After the washing steps with PBS the pellet was resuspended in 400 µL PBS and transferred in FACS tubes (Falcon ^{®} Round-Bottom Tubes with cell strainer cap; Corning). The measurement was performed with a FACS Canto II and the data were analyzed by the software FlowJo.

### Example 5

### Fed-batch cultivation

Fed-batch production cultures were performed in shake flasks or Ambr15 vessels (Sartorius Stedim) with proprietary chemically defined medium. Cells were seeded at 1x10E6 cells/ml on day 0, with a temperature shift on day 3. Cultures received proprietary feed medium on days 3, 7, and 10. Viable cell count (VCC) and percent viability of cells in culture was measured on days 0, 3, 7, 10, and 14 using a Cedex HiRes instrument (Roche Diagnostics GmbH, Mannheim, Germany). Glucose, lactate and product titer concentrations were measured on days 3, 5, 7, 10, 12 and 14 using a Cobas Analyzer (Roche Diagnostics GmbH, Mannheim, Germany). The supernatant was harvested 14 days after start of fed-batch by centrifugation (10 min, 1000 rpm and 10 min, 4000 rpm) and cleared by filtration (0.22 µm). Day 14 titers were determined using protein A affinity chromatography with UV detection. Product quality was determined by Caliper's LabChip (Caliper Life Sciences).

### Example 6

### RNP-based CRISPR-Cas9 gene knock-outs in CHO cells

### Material/Resources:

- Geneious 11.1.5 for guide and primer design
- CHO TI host cell line; cultivation state: day 30-60
- TrueCut^{™} Cas9 Protein v2 (Invitrogen^{™})
- TrueGuide Synthetic gRNA (custom designed against target gene, 3nm unmodified gRNA, Thermo Fisher)
- TrueGuide^{™} sgRNA Negative Control, non-targeting 1 (Thermo Fisher)
- medium (200 µg/ml Hygromycin B, 4 µg/ml selection agent 2)
- DPBS - Dulbecco's Phosphate-Buffered Saline w/o Ca and Mg (Thermo Fisher)
- Microplate 24 deep well plate (Agilent Technologies, Porvoir science) with cover (self-made)
- Thin, long RNase, DNase, pyrogen free filter tips for loading OC-100 cassettes. (Biozyme)
- Hera Safe Hood (Thermo Fisher)
- Cedex HiRes Analyzer (Innovatis)
- Liconic Incubator Storex IC
- HyClone electroporation buffer
- MaxCyte OC-100 cassettes
- MaxCyte STX electroporation system

### CRISPR-Cas9 RNP delivery

RNPs are preassembled by mixing 5 µg Cas9 with 1 µg gRNA mix (equal ratio of each gRNA) in 10 µL PBS and incubated for 20 minutes at RT. Cells with a concentration between 2-4x10E6 cell/mL are centrifuged (3 minutes, 300 g) and washed with 500 µL PBS. After the washing step, the cells are again centrifuged (3 minutes, 300 g) and resuspended in 90 µL Hyclone electroporation buffer. The preincubated RNP mix is added to the cells and incubated for 5 minutes. The cell/RNP solution is then transferred into an OC-100 cuvette and electroporated with program "CHO2" using a MaxCyte electroporation system. Immediately after electroporation, the cell suspension is transferred into a 24 dwell and incubated at 37°C for 30 minutes. Fresh and pre-warmed medium is added to a final cell concentration of 1x10E6 and incubated at 37°C and 350 rpm for cell expansion. For genomic DNA preparation (day 6 or 8), QuickExtract kit (Lucigen) was added to the cells and served as a PCR template. Specific SIRT-1 amplicon was PCR-amplified using standard Q5 Hot Start Polymerase protocol (NEB) and primer oSA060 and oSA061 (SEQ ID NO: 15 and 16). Amplicon was purified using QIAquick PCR purification kit (Quiagen) and analyzed by Sanger sequencing by Eurofins Genomics GmbH.

### Fed-batch cultivation

Fed-batch production cultures were performed in shake flasks or Ambr15 vessels (Sartorius Stedim) with proprietary chemically defined medium. Cells were seeded at 1x10E6 cells/ml. Cultures received proprietary feed medium on days 3, 7, and 10. Viable cell count (VCC) and percent viability of cells in culture was measured on days 0, 3, 7, 10, and 14 using a Cedex HiRes (Roche Diagnostics GmbH, Mannheim, Germany). Glucose, lactate and product titer concentrations were measured on days 3, 5, 7, 10, 12 and 14 using a Cobas analyzer (Roche Diagnostics GmbH, Mannheim, Germany). The supernatant was harvested 14 days after start of fed-batch by centrifugation (10 min, 1000 rpm and 10 min, 4000 rpm) and cleared by filtration (0.22 µm). Day 14 titers were determined using protein A affinity chromatography with UV detection. Product quality was determined by Caliper's LabChip (Caliper Life Sciences).

## Claims

1. A mammalian cell wherein the activity or function or expression of the endogenous SIRT-1 gene has been reduced or eliminated or diminished or completely knocked-out.

2. A mammalian cell wherein the expression of the endogenous SIRT-1 gene has been reduced and wherein said mammalian cell has at least one of increased productivity for heterologous polypeptides and/or reduced lactate production during cultivation and/or extended high viability levels during cultivation and/or extended cultivation time compared to a cell cultivated under the same conditions that has the identical genotype but endogenous SIRT-1 gene expression.

3. The mammalian cell according to any one of claims 1 to 2, wherein the SIRT-1 gene knockout is a heterozygous knockout or a homozygous knockout.

4. The mammalian cell according to any one of claims 1 to 3, wherein the productivity of the SIRT-1 knockout cell is at least 10 % increased compared to a SIRT-1 expressing parent mammalian cell.

5. The mammalian cell according to any one of claims 1 to 4, wherein the reduction or elimination or diminishment or knockout is mediated by a nuclease-assisted gene targeting system.

6. The mammalian cell according to claim 5, wherein the nuclease-assisted gene targeting system is selected from the group consisting of CRISPR/Cas9, CRISPR/Cpf1, zinc-finger nuclease and TALEN.

7. The mammalian cell according to any one of claims 1 to 4, wherein the reduction of SIRT-1 gene expression is mediated by RNA silencing.

8. The mammalian cell according to claim 7, wherein the RNA silencing is selected from the group consisting of siRNA gene targeting and knock-down, shRNA gene targeting and knock-down, and miRNA gene targeting and knock-down.

9. The mammalian cell according to any one of claims 2 to 8, wherein the SIRT-1 knockout is performed before the introduction of the exogenous nucleic acid encoding the heterologous polypeptide or after the introduction of the exogenous nucleic acid encoding the heterologous polypeptide.

10. The mammalian cell according to any one of claims 2 to 9, wherein the polypeptide is an antibody.

11. The mammalian cell according to claim 10, wherein the antibody is a multispecific antibody.

12. The mammalian cell according to any one of claims 10 to 11, wherein the multispecific antibody is selected from the group consisting of
i) a full-length antibody with domain exchange comprising a first Fab fragment and a second Fab fragment,
wherein in the first Fab fragment
a) the light chain of the first Fab fragment comprises a VL and a CH1 domain and the heavy chain of the first Fab fragment comprises a VH and a CL domain;
b) the light chain of the first Fab fragment comprises a VH and a CL domain and the heavy chain of the first Fab fragment comprises a VL and a CH1 domain; or
c) the light chain of the first Fab fragment comprises a VH and a CH1 domain and the heavy chain of the first Fab fragment comprises a VL and a CL domain; and
wherein the second Fab fragment comprises a light chain comprising a VL and a CL domain, and a heavy chain comprising a VH and a CH1 domain;
ii) a full-length antibody with domain exchange and additional heavy chain C-terminal binding site comprising
- one full length antibody comprising two pairs each of a full length antibody light chain and a full length antibody heavy chain, wherein the binding sites formed by each of the pairs of the full length heavy chain and the full length light chain specifically bind to a first antigen; and
- one additional Fab fragment, wherein the additional Fab fragment is fused to the C-terminus of one heavy chain of the full length antibody, wherein the binding site of the additional Fab fragment specifically binds to a second antigen;
wherein the additional Fab fragment specifically binding to the second antigen i) comprises a domain crossover such that a) the light chain variable domain (VL) and the heavy chain variable domain (VH) are replaced by each other, or b) the light chain constant domain (CL) and the heavy chain constant domain (CH1) are replaced by each other, or ii) is a single chain Fab fragment;
iii) a one-armed single chain antibody comprising a first binding site that specifically binds to a first epitope or antigen and a second binding site that specifically binds to a second epitope or antigen, comprising
- a light chain comprising a variable light chain domain and a light chain kappa or lambda constant domain;
- a combined light/heavy chain comprising a variable light chain domain, a light chain constant domain, a peptidic linker, a variable heavy chain domain, a CH1 domain, a Hinge region, a CH2 domain, and a CH3 with knob mutation;
- a heavy chain comprising a variable heavy chain domain, a CH1 domain, a hinge region, a CH2 domain, and a CH3 domain with hole mutation;
iv) a two-armed single chain antibody comprising a first binding site that specifically binds to a first epitope or antigen and a second binding site that specifically binds to a second epitope or antigen, comprising
- a first combined light/heavy chain comprising a variable light chain domain, a light chain constant domain, a peptidic linker, a variable heavy chain domain, a CH1 domain, a Hinge region, a CH2 domain, and a CH3 with hole mutation;
- a second combined light/heavy chain comprising a variable light chain domain, a light chain constant domain, a peptidic linker, a variable heavy chain domain, a CH1 domain, a Hinge region, a CH2 domain, and a CH3 domain with knob mutation;
v) a common light chain bispecific antibody comprising a first binding site that specifically binds to a first epitope or antigen and a second binding site that specifically binds to a second epitope or antigen, comprising
- a light chain comprising a variable light chain domain and a light chain constant domain;
- a first heavy chain comprising a variable heavy chain domain, a CH1 domain, a Hinge region, a CH2 domain, and a CH3 domain with hole mutation;
- a second heavy chain comprising a variable heavy chain domain, a CH1 domain, a Hinge region, a CH2 domain, and a CH3 domain with knob mutation;
vi) a full-length antibody with additional heavy chain N-terminal binding site with domain exchange comprising
- a first and a second Fab fragment, wherein each binding site of the first and the second Fab fragment specifically bind to a first antigen;
- a third Fab fragment, wherein the binding site of the third Fab fragment specifically binds to a second antigen, and wherein the third Fab fragment comprises a domain crossover such that the variable light chain domain (VL) and the variable heavy chain domain (VH) are replaced by each other; and
- an Fc-region comprising a first Fc-region polypeptide and a second Fc-region polypeptide;
wherein the first and the second Fab fragment each comprise a heavy chain fragment and a full length light chain,
wherein the C-terminus of the heavy chain fragment of the first Fab fragment is fused to the N-terminus of the first Fc-region polypeptide,
wherein the C-terminus of the heavy chain fragment of the second Fab fragment is fused to the N-terminus of the variable light chain domain of the third Fab fragment and the C-terminus of the CH1 domain of the third Fab fragment is fused to the N-terminus of the second Fc-region polypeptide;
and
vii) an immunoconjugate comprising a full-length antibody and a non-immunoglobulin moiety conjugated to each other optionally via a peptidic linker.
